(19) **Europäisches Patentamt / European Patent Office / Office européen des brevets**

(11) **EP 1 661 906 B1**

(12) # EUROPEAN PATENT SPECIFICATION

(45) Date of publication and mention
of the grant of the patent:
**04.01.2012 Bulletin 2012/01**

(21) Application number: **04787627.1**

(22) Date of filing: **06.09.2004**

(51) Int Cl.:
*C07K 1/22* (2006.01)     *C07K 1/34* (2006.01)
*C07K 1/36* (2006.01)     *C12P 1/00* (2006.01)
*G01N 33/68* (2006.01)     *C12M 1/00* (2006.01)

(86) International application number:
**PCT/JP2004/012923**

(87) International publication number:
**WO 2005/028500 (31.03.2005 Gazette 2005/13)**

(54) **METHOD OF PREPARING SOLUTION FOR PROTEOMIC ANALYSIS**

VERFAHREN ZUR HERSTELLUNG EINER PROTEOMANALYSE-LÖSUNG

PROCÉDÉ DE PRÉPARATION D'UNE SOLUTION POUR L'ANALYSE PROTÉOMIQUE

(84) Designated Contracting States:
**AT BE BG CH CY CZ DE DK EE ES FI FR GB GR HU IE IT LI LU MC NL PL PT RO SE SI SK TR**

(30) Priority: **05.09.2003 JP 2003313567**
**21.01.2004 JP 2004013257**

(43) Date of publication of application:
**31.05.2006 Bulletin 2006/22**

(73) Proprietor: **TORAY INDUSTRIES, INC.**
**Tokyo 103-8666 (JP)**

(72) Inventors:
• **WADA, Shigehisa**
**5200842 (JP)**
• **UTSUMI, Jun**
**1310033 (JP)**
• **FUJITA, Yoshiji**
**1510071 (JP)**
• **SUGAYA, Hiroyuki**
**5202101 (JP)**
• **YAMADA, Satoko**
**5200854 (JP)**

(74) Representative: **Webster, Jeremy Mark et al**
**Mewburn Ellis LLP**
**33 Gutter Lane**
**London**
**EC2V 8AS (GB)**

(56) References cited:
EP-A- 0 232 885     EP-A- 0 344 581
EP-A- 0 750 938     WO-A2-01/72844

JP-A- 01 022 259     JP-A- 01 230 370
US-A1- 2003 132 114

• **RADHAKRISHNA S TIRUMALAI ET AL.: "Characterization of the Low Molecular Weight Human Serum Proteome" MOLECULAR & CELLULAR PROTEOMICS, vol. 2, no. 10, 13 August 2003 (2003-08-13), pages 1096-1103, XP002390543**
• **HIGUCHI A ET AL: "Serum protein adsorption and platelet adhesion on pluronic(TM)-adsorbed polysulfone membranes" BIOMATERIALS, ELSEVIER SCIENCE PUBLISHERS BV., BARKING, GB, vol. 24, no. 19, August 2003 (2003-08), pages 3235-3245, XP004425368 ISSN: 0142-9612**
• **GOVORUKHINA N I ET AL: "Sample preparation of human serum for the analysis of tumor markers - Comparison of different approaches for albumin and gamma-globulin depletion" JOURNAL OF CHROMATOGRAPHY A, ELSEVIER, AMSTERDAM, NL, vol. 1009, no. 1-2, 15 August 2003 (2003-08-15), pages 171-178, XP004447859 ISSN: 0021-9673**
• **AHMED N. ET AL.: 'An approach to remove albumin for the proteomic analysis of low abundance biomarkers in human serum' PROTEOMICS vol. 3, no. 10, 23 June 2003, pages 1980 - 1987, XP002986338**
• **ROTHEMUND D.L. ET AL.: 'Depletion of the highly abundant protein albumin from uman plasma using the gradiflow' PROTEOMICS vol. 3, no. 3, March 2003, pages 279 - 287, XP002986339**

Remarks:
The file contains technical information submitted after the application was filed and not included in this specification

**Description**

The invention relates to a method and an apparatus of preparing a solution containing biological components, particularly a solution containing biological components with changed composition obtained by isolating biological molecules of such as proteins extracted from human serum, urine, or the like. Specially, the invention relates to a method and an apparatus for the preparation of a solution of biological components with changed composition by removing components inhibiting detection of trace components, particularly removing proteins with high molecular weights for the purpose of carrying out clinical proteome analysis

[Background of the Art]

Recently, proteome analysis research (proteomics) has begun to draw attention as postgenome research. Since it is a very likely supposition that proteins, gene products, are more directly linked with syndromes of diseases than genes, it has been highly expected that research findings and achievements of proteome analysis of thoroughly investigating proteins can widely be applicable for diagnosis and medical care. Moreover, it is highly possible to find many proteins causing diseases and factors relevant to diseases, which cannot be found by genome analysis.

High speed structural analysis is made possible by MS (mass spectrometry) and technically it has greatly contributed to rapid advancement of proteome analysis and practical application of MALDI-TOF-MS (matrix assisted laser desorption ionization time-of-flight mass spectrometry) has enabled ultramicroanalysis of polypeptides to be performed at a high throughput, and that makes it possible to identify even trace proteins which have not be detected conventionally and accordingly becomes a powerful tool for searching factors relevant to diseases.

The first purpose of the clinical application of proteome analysis is to find biomarker proteins induced or eliminated by diseases. The biomarker behaves in relation to symptoms of diseases, so that it can be a marker for diagnosis and also highly possibly becomes a target for producing pharmaceuticals. That is, since the findings and achievements of proteome analysis are highly possibly applicable to find a diagnosis marker and a target for producing pharmaceuticals rather than specified gene, it can be said that proteome analysis becomes a key technology for diagnosis and medical care in the postgenome age and since the identified biomarker directly brings profits to patients, that is, evaluation of response to the pharmaceuticals and expectation of side effect development, it can be said that this technique plays an important role to promote so-called tailor-made medical care.

In case proteome analysis is to be introduced in clinical research, it is necessary to quickly and reliably analyze a large number of samples and moreover, since each clinical sample is slight in the amount and very precious, it is required to carry out high resolution, high sensitivity, and highly functional measurement. Mass spectrometry has considerably propelled the analysis, and the characteristics of mass spectrometers, that is, high sensitivity and high throughput, have greatly contributed to the analysis. However, although the techniques and appliances have been improved swiftly, the present situation is not yet ready to simply and quickly carry out proteome analysis in a clinical field.

One of the causes is attributed to the pretreatment of clinical samples. It is needed to fractionate and refine proteins of a clinical sample before mass analysis, and the treatment still takes several days, and the operation of the pretreatment is complicated and requires experiences and skills and that becomes a high obstacle to clinical application. If diagnosis of a disease in the entire body and symptom control are made possible with a small amount of blood and body fluid, it is remarkably useful. However, there are many challenges to overcome, due to the variation of proteins contained in blood plasma.

It is assumed that there are 100, 000 or more kinds of human proteins and about 10,000 kinds of proteins are contained in serum and the concentration of the total proteins in the serum is about 60 to 80 mg/mL. The proteins contained in a human serum are albumin (molecular weight: 66 kDa), immunoglobulin (150 to 190 kDa), transferrin (80 kDa), haptoglobin (>85 kDa), and lipoprotein (several 100kDa) and all of them exist respectively in an amount exceeding 1 mg/mL. On the other hand, many of physiological active proteins such as peptide hormones, interleukin, and cytokine regarded to be biomarkers of symptoms and factors relevant to diseases exist in a trace lower than 1 ng/mL and the contents are no more than nano to pico level as compared those of the high content components with high molecular weights. In terms of the size of proteins, 70% or less in all kinds of proteins have a molecular weight of 60,000 (60kDa) or lower and the above-mentioned biomarker proteins existing in a trace are almost all included in this range (reference to Non-patent Document No.1). Since these proteins are partially excreted to urine through the kidneys not only blood but also urine may be used as a sample.

To carry out proteome analysis by general serologic investigation, it is at first essential to remove high molecular weight components with a molecular weight of 60,000 or higher, which become obstacles to detection of trace components relevant to pathogenic causes, and recover proteins with a molecular weight less than 15,000 as much as possible.

Presently, high performance liquid chromatography (LC) and 2-dimensional electrophoresis (2 dimensional-polyacrylamide gel electrophoresis: 2D-PAGE) have been employed as means of separation and removal of the high molecular weight proteins, however it takes 1 to 2 days only for LC and 2D-PAGE operation. The time needed for them is very long as compared with the analysis time, several minutes, for MALDI-TOF-MS and ESI-MS (electrospray ionization mass spectrometry) and the remarkable advantageous point that MS, an analysis means, has a high throughput cannot sufficiently be exhibited in clinical proteome analysis. Therefore, it must be said that at the present moment, MS is insufficient in practical applications for the purpose of obtaining analysis results within a time as short as possible for diagnosis and medical care in medical treatment fields and it becomes a significant cause of difficulty of utilization of MS for the daily clinical investigations.

Therefore, it is expected that promptness of diagnosis of the clinical investigations by clinical proteome analysis may remarkably be improved if the means of removing a portion or all of high molecular weight proteins from a sample is accelerated. Practically, that can be accomplished if a method or an apparatus of obtaining a biological components-containing solution with a changed composition of biological components while leaving a group of aimed proteins from a small amount of a sample at a high speed are made available in place of LC and 2D-PAGE.

As already practically utilized products or disclosed techniques for means of removing a main object substance, albumin, there are a carrier (commercialized) in which an affinity ligand such as a blue dye is immobilized, a centrifugal tubular apparatus (commercialized) for fractionating the high molecular weight components by centrifugal filtration, a method of fractionation by electrophoresis principle, a traditional precipitation method such as ethanol precipitation by Cohn, and a method of fractionation by chromatography (reference to Non-patent Document No. 2).

However, they all have problems such as insufficiency of the separation capability, unsuitability for a very small amount of a sample, and contamination of chemical agents, which are obstacles for mass spectrometry. Particularly, a method of removing albumin as a target solely by adsorption is capable of removing albumin, however it is difficult for the method to remove the high molecular weight components with a molecular weight of 60,000 or higher such as immunoglobulin.

Separation membranes with various sizes in accordance with the utilization have been developed and employed for an artificial kidney, an artificial lung, and a plasma separator and also improved for heightening the affinity with biological components (reference to Patent Document No. 2). However there is no implication of solutions to the problems of the clinical proteomics that the high molecular weight proteins such as albumin have to be removed at a high efficiency.

If a method or an apparatus capable of solving these problems could be developed, proteome analysis would be performed widely in medical researches and clinical work fields and it is made possible to carry out investigation and diagnosis at a higher speed and a higher precision and accordingly, the method or the apparatus is expected to be a powerful tool to investigate causes of hardly curable diseases for which efficacious medical caring methods are not yet available, or develop methods of diagnosing these diseases in early stage.

As described above, it is needed to remove excess high molecular weight proteins which are obstacles in clinical proteome analysis. It has been required so far to develop a device having a high separation capability more convenient and faster than techniques such as 2D-PAGE and liquid chromatography which are complicated and take a long time.

In these years, a method of using a gel, Affi-Gel Blue, (reference to Non-patent Document No. 3) and a method of using "Gradiflow" system (reference to Non-patent Document No. 4) are reported as effective and improved albumin removal methods, however they are not yet capable of preparing solutions for analysis to obtain much more information.

The conditions required for the techniques of removal of albumin from blood plasma are that the components of blood plasma are passed at a high speed; that there is not protein denaturation function; that very fine processing is done for high functionality; and that they are not considerably costly. Neither apparatus nor device that can solve the above-mentioned problems and satisfy the conditions has made available yet.

[Non-patent Document No.1] Anderson NL, Anderson NG, "The human plasma proteome: history, character, and diagnostic prospects)", proteomics(Molecular & Cellular Proteomics), USA, The American Society for Biochemistry and Molecular Biology, Inc. , (2002) vol. 1, p845-867.

[Non-patent Document No. 2] The Japanese Biochemical Society, "New Biochemical Experiments, vol. 1; Proteins (1) separation · refining · characteristics", TOKYO KAGAKUDOZIN CO., LTD. (1990[Non-patent Document No. 3] CELL TECHNOLOGY, special edition, "Illustrated Bioexperiment 5", SHUJUNSHA Co., Ltd. (2001)

[Non-patent Document No. 4] N. Ahmed et al., Proteomics, On-line issue, June 23, 2003

[Non-patent Document No. 5] D. L. Rothemund et al. (2003), Proteomics, vol. 3, p279-287

[Patent Document No. 1] Japanese Patent Application National Publication (Laid-Open) No. 2002-542163

[Patent Document No. 2] Japanese Patent No. 3297707

[Disclosure of the Inventions]

[Problems to be Solved by the Inventions]

In view of the above state of the art, it is an object of the invention to provide a method and an apparatus of preparing a biological components-containing solution with a composition of changed biological components suitable for proteome analysis by separating and removing excess high molecular weight proteins to be obstacles at the time of clinical proteome analysis from a biological components-containing solution.

Accordingly, in a first aspect the invention provides a method of preparing a solution for proteome analysis having a composition of biological components changed to control the concentration ratio of albumin in the total proteins to be less than 0.3 and the concentration ratio of β2-microglobulin in the total proteins in the solution having a changed composition of biological components is at least 10 times as high as the composition ratio of β2-microglobulin in the total proteins in the biological components-containing solution supplied to the membrane, as defined in the accompanying claim 1.

The comparative permeation ratio of β2-microglobulin to albumin is defined as:

sieving coefficient of β2-microglobulin,
sieving coefficient of albumin

Embodiments of this aspect of the invention concern the following:

1. The method of preparing the above-mentioned solution wherein the above-mentioned separation membrane has a 70 or higher comparative permeation ratio.
2. The method of preparing either one of the above-mentioned solutions wherein the concentration ratio is less than 0.1.
3. The method of preparing the above-mentioned solution wherein the ratio is at least 100 times as high.
4. The method of preparing the above-mentioned solution wherein the biological components are a solution containing proteins extracted from substances derived from blood, blood plasma, serums, urine, ascites, saliva, tear, cerebrospinal fluid, pleural exudate, or cells.

In a further aspect the invention provides a method of analysis of proteins contained in biological components by preparing a solution having a changed composition of the biological components by one of the methods described above and then analyzing the proteins contained in the solution.

In the present method of analysis of proteins, the means of analyzing proteins may be at least one selected from mass spectrometry, electrophoretic analysis, and liquid chromatography.

Another aspect of the invention concerns an apparatus for preparing a solution for proteome analysis having a changed composition of biological components in which the concentration ratio of albumin in the total proteins is less than 0.3 and the concentration ratio of β2-microglobulin in the total proteins is at least 10 times as high as the concentration ratio in the biological components-containing starting solution, which apparatus is as defined in the accompanying claim 13.

Embodiments of this aspect may include apparatus which contains a liquid flow channel comprising a module containing a polysulfone separation membrane which contains flow channels having an asymmetric structure and which has a 50 or higher comparative permeation ratio of β2-microglobulin to albumin and the module has a raw liquid inlet and a raw liquid outlet joined to a raw liquid side flow path of the separation membrane; a solution circulation channel communicating the raw liquid inlet and the raw liquid outlet and having a pump and an inlet for an object solution for separation in the middle; and a diluent solution inlet formed at a position upstream of the inlet for an object solution for separation or a position in the middle of the solution.

Embodiments of this aspect of the invention concern an apparatus for preparing a solution having a changed composition of biological components; wherein the apparatus comprises at least two liquid flow channels as described above; wherein the outlet of the object solution for separation of one liquid flow channel is joined directly or indirectly to an inlet for the object solution for separation of the other liquid flow channel.

Embodiments of the invention may provide a method of preparing a solution having a changed composition of biological components for proteome analysis in which the concentration ratio of albumin in the total proteins is less than 0.3 and the concentration ratio of β2-microglobulin in the total proteins is at least 10 times as high as the concentration ratio in the biological components-containing starting solution by introducing a biological components-containing solution in the raw liquid side of a module containing a polysulfone separation membrane which contains flow channels having an asymmetric structure and which has a comparative permeation ratio of β2-microglobulin to albumin of at least 50, circulating the solution in the raw liquid side through a solution formed in the outside of the module, and taking out the solution passed through the separation membrane as the solution having a changed composition of biological components; wherein a diluting solution for the biological components-containing solution is additionally introduced into the raw liquid side of the separation membrane disposed in the module immediately after introduction of the biological components-containing solution, and the separation is carried out under the condition satisfying $0 < Q2/Q1 < 1$ wherein Q1 denotes the flow speed of the biological components-containing solution introduced into the raw liquid side of the separation membrane and Q2 denotes the flow speed of the solution passed through the separation membrane

In embodiments of this method of preparing a solution Q2/Q1 may satisfy $0.005 \leq Q2/Q1 \leq 0.5$.

In further embodiments of the method of preparing the solution as described in one of the above-mentioned methods the flow speed Q2 of the passed solution and the total flow speed Q3 of the biological components-containing solution introduced into the raw liquid side and the diluting solution satisfies $0.5 \leq Q2/Q3 \leq 1.5$.

In the method of preparing a solution as described above; Q2/Q3 may be about 1.

In the method of preparing a solution as described in one of the above-mentioned methods; a physiological salt solution or a buffer solution may be used as the diluting solution.

In embodiments of the method of preparing a solution as described in one of the above-mentioned methods; the biological components are a solution containing proteins extracted from substances derived from blood, blood plasma, serums, urine, ascites, saliva, tear, cerebrospinal fluid, pleural exudate, or cells.

In further embodiments of the method of preparing a solution having a changed composition of biological components as described in one of the above-mentioned methods; two modules are employed and a solution obtained by either one of the methods described above is used in a first module and either one of the methods is carried out by the second module.

An additional aspect of the invention provides a method of preparing a solution for proteome analysis having a changed composition of biological components in which the concentration ratio of albumin in the total proteins is less than 0.3 and the concentration ratio of β2-microglobulin in the total proteins is at least 10 times as high as the concentration ratio in the biological components-containing starting solution, the method comprising subjecting the biological components-containing solution to treatment in at least two steps; characterized in that the two steps are carried out in combination and selected from (1) a step of adsorbing a portion or all of proteins having a molecular weight equal to or higher than that of albumin; (2) a step of removing a portion or all of proteins having a molecular weight equal to or higher than that of albumin by fractionation with a molecular sieve; and (3) a step of concentrating proteins, wherein a polysulfone hollow fiber separation membrane containing flow channels having an asymmetric structure and which is derived from polysulfone and poly(vinyl pyrrolidone) and has a comparative permeation ratio of β2-microglobulin to albumin of at least 50 is employed in step (1) or step (2).

In embodiments of the method of preparing a solution as described in one of the above-mentioned method a separation membrane containing one or more substances selected from cellulose, cellulose acetate, a polycarbonate, a polysulfone, a poly(methacrylic acid) ester, a poly(acrylic acid) ester, a polyamide, polyvinylidene fluoride, polyacrylonitrile, polyethylene, and polypropylene is used in the step (3).

In further embodiments the method of preparing a solution as described in one of the above-mentioned methods, a material fixing one or more substances selected from a group consisting of a polyethylene imine, an aminomethylpyridine, a polyphenol, a blue dye, a divalent metal ion, and an alkyl group-containing compound in the surface is used in the step (1) or the step (2).

In additional embodiments of the method of preparing a solution as described in one of the above-mentioned methods; one or more substances selected from a group consisting of a surfactant, an emulsifier, an organic solvent, an alcohol, an ethylene glycol, a propylene glycol, a polyethylene imine, an aminomethylpyridine, protamine sulfate, ammonium sulfate, a polyphenol, a blue dye, a caotropic salt, and an alkyl-containing compound are added to an aqueous solution in the step (1) or the step (2).

In a method of preparing a solution as described in one of the above-mentioned methods the biological components-containing solution may contain a sample of human-derived components.

In the second aspect the invention provides an apparatus for preparing a solution suitable for proteome analysis having a changed composition in which the concentration ratio of albumin in the total proteins is less than 0.3 and the concentration of β2-microglobulin in the total proteins is at least 10 times as high as the concentration ratio in the starting solution wherein the apparatus comprises at least two kinds of means joined by a flow path and selected from (1) means of adsorbing a portion or all of proteins having a molecular weight equal to or higher than that of albumin; (2) means of removing a portion or all of proteins having a molecular weight equal to or higher than that of albumin by fractionation with a molecular sieve; and (3) means of concentrating proteins and wherein (1) or (2) comprises a polysulfone hollow fiber separation membrane containing flow channels having an asymmetric structure which is derived from polysulfone and poly(vinyl pyrrolidone) and has a comparative permeation ratio of β2-microglobulin to albumin of at least 50.

Embodiments of such apparatus for preparing a solution as described above may comprise a liquid flow-out path to be joined to a liquid chromatograph, an electrophoretic apparatus, or a mass spectrometer.

[Effects of the Inventions]

The methods and apparatuses disclosed herein make it possible to prepare a solution within a short time with which many trace proteins which have conventionally been difficult to be detected from biological components such as blood, serum, and blood plasma.

[Brief Description of Drawings]

[Fig. 1] A schematic view of a separation system used for Examples 1 and 3 of the invention (one membrane separation unit).

[Fig.2] A conceptual view of a separation system used for Example 2 of the invention (two membrane separation units).

[Fig. 3] A schematic view of a separation system used for Example 4 of the invention (three membrane separation units).

[Fig. 4] A schematic view of a separation system used for Examples 5 and 6 of the invention (example comprising three units having membrane separation function and adsorption function and one concentration unit).

[Fig. 5] A schematic view of one example of the apparatus illustrating the invention.

[Fig. 6] A photograph of electrophoresis of a solution with a changed composition of biological components.

[Fig. 7] A photograph of two-dimensional electrophoresis of a solution with a changed composition of biological components obtained in Example 2.

[Fig. 8] A photograph of two-dimensional electrophoresis of a human serum solution.

[Explanation of Symbols]

1 a valve
2 a solution circulation channel
3 a pump for circulation
4 a treatment solution recovery port
5 a module for the first step
6 a module for the second step
7 a module for the third step
8 a pump for injection
100 a pump for injection
101 a three-way valve
102 a solution circulation channel
103 a pump for circulation
104 a recovery port of a membrane separation unit-treated solution
105 a separation membrane module
106 a lower port of a module
202 a second-step solution circulation channel
203 a second-step pump for circulation
204 a second-step recovery port of a membrane separation unit-treated solution
205 a second separation membrane module
206 a lower port of a second-step module
302 a third-step solution circulation channel
303 a third-step pump for circulation
304 a third-step recovery port of a membrane separation unit-treated solution
305 a third separation membrane module
306 a lower port of a third-step module
402 a solution of a concentration unit circulation channel
403 a circulation pump of a concentration unit
404 a filtrate outlet of a concentration unit
405 a concentration membrane module
406 a lower port of a module for concentration
407 a treatment solution recovery port of a concentration unit

[Best Mode of Embodiments of the Inventions]

At first common parts in the inventions will be described.

"A solution with a changed composition of biological components" in the inventions means a solution obtained by treating a biological components-containing solution that is a living body-derived solution such as blood as a raw solution by a specified treatment and thereby changing the composition of proteins. "Blood" herein means blood of animals, e.g. human being, and includes solutions containing parts of components, e.g. serum and blood plasma, in blood. "A solution derived from a living body" includes a solution containing substances, e.g. proteins, relevant to a living body and a solution obtained by extracting proteins from blood as well as urine, saliva, tear, cerebrospinal fluid, ascites, pleural exudate, or cells can be exemplified.

"A separation membrane module" in the invention comprises a separation membrane stored in a housing. The housing has an inlet through which a solution to be separated is introduced and an outlet through which a separated solution passed through the separation membrane is discharged. The material of the housing is not particularly limited and a housing made of a plastic such as a polycarbonate, polypropylene, polystyrene, a polysulfone, and a polyether sulfone can be exemplified.

The solution obtained according to the inventions by changing the composition of a biological components-containing solution is preferably used for protein analysis, particularly proteome analysis. The analysis method is not particularly limited and LC, 2D-PAGE, nuclear magnetic resonance (NMR), MALDI-TOF-MS, and ESI-MS can be exemplified. These methods are analysis methods whose analysis sensitivity is lowered if the solution contains a high content of proteins such as albumin, which exist in a large quantity in some of solutions, and use of the solution obtained according to the invention makes highly sensitive analysis possible. Also, the solution is advantageous for proteome analysis using MS or electrophoresis. MS to which a solution preparation apparatus of the inventions is directly or indirectly joined is not particularly limited and preferably an electrospray ionization type, an atmospheric pressure ionization type, a quadrupole (QQQ) type, a magnetic sector type, a time-of-flight type, MS/MS, MSn, FT-MS type, an ion capture type, and their combination type can be exemplified. Further, the apparatus may also include a tandem MS just like MS/MS and MSn (e.g. MS3). In the case of the tandem MS, all types of MS are usable and particularly, combination use of sector appliances, e.g. ion capture type, quadrupole-time-of-flight type (Q-TOF), FT-MS, and quadrupole and ion capture gives a high efficiency. Accordingly, it is made possible to selective detection of peaks generated in MS/MS and/or MSn measurement.

Structural information of various kinds of trace protein components can be collected if a solution obtained by the methods or refining apparatuses of the inventions and the above-mentioned analysis apparatuses are employed and further developments are performed. The information includes not only peptide-mass fingerprint (PMF) but also primary structure information (aminoacid sequences) of respective peptides.

Next, the invention will be described.

In the the invention, to obtain a solution with a changed composition of biological components and having a concentration ratio of specified albumin of lower than 0.3, a method involving steps of supplying a biological components-containing solution to a separation membrane having a comparative permeation ratio, which is a value calculated by dividing a sieving coefficient of β2-microglobulin by that of albumin, of 50 or higher and collecting the solution passed through the separation membrane is employed. For the method, an apparatus having a module which contains a separation membrane having a comparative permeation ratio of β2-microglobulin to albumin of 50 or higher and which has a raw liquid inlet for the biological components-containing solution in the raw liquid side of the separation membrane and an outlet of the filtrate passed through the separation membrane is used. In the module of the above-mentioned apparatus, the module may have a raw liquid outlet for the biological components-containing solution in the raw liquid side of the separation membrane.

The comparative permeation ratio of the separation membrane is preferably 70 or higher and more preferably 140 or higher, and although there is no particular upper limit, however if its value is so high, the recovery amount of desired proteins with a molecular weight lower than 15, 000 may possibly be lowered and therefore, it is preferably 10,000 or lower. A hollow fiber membrane is selected to be used as the separation membrane. For the purpose of filtering proteins, many hollow fiber membranes have been employed as materials of dialysis modules conventionally called as an artificial kidney. The separation membranes to be used for the artificial kidney are so designed as to prevent proteins such as albumin from passing and permeate low molecular weight components such as creatinine and urea as much as possible and accordingly is employed for purifying blood by leading blood to the inside of hollow fibers and discharging low molecular weight proteins unnecessary for a living body out of the outside of hollow fibers. In the separation membrane of the invention, a technique of passing an object solution from the inside of hollow fibers to the outside through the hollow fiber membrane is preferable. Particularly, a method of obtaining a solution by preventing high molecular weight components such as albumin from passing in the inside of the hollow fibers and on the other hand allowing mainly proteins with a molecular weight of 15, 000 to pass the hollow fibers is preferable. Although similar results can be obtained even if a plane membrane type separation membrane is used, utilization of the hollow fiber membrane makes it easy to enlarge the surface area per treatment solution amount and lowers the pressure loss in the operation, resulting in efficient execution of the inventions.

The material of the separation membrane to be used in the invention is not particularly limited and usable materials are those containing one or more kinds of polymers selected from a group consisting of polymers such as polysulfones and polyether sulfones. Among them, polysulfones widely used in these years for dialyzers are preferable materials because of an excellent fractionating property of the polysulfones. In terms of the membrane structure, the flow channels in the inside of the separation membrane have an asymmetric structure and preferably have a multilayer asymmetric structure composed of a dense layer and a support layer having a high porosity and keeping the membrane strength. The asymmetric structure is judged by observation of cross-sectional structure of the membrane with an electron microscope under the observation condition of 1,000 times magnification. The structure is judged based on whether there are both of a layer in which voids are scarcely observed and a layer in which voids are observed in the thickness direction of the membrane. If existence of both layers are observed, the asymmetric structure is certified.

In the asymmetric structure, it is preferable that the voids existing near the face with which a raw solution is to be brought into contact are densest. That is because blocking in the membrane surface with solutes can be suppressed.

It is preferable for the separation membrane to be used for the invention to absorb proteins as scarcely as possible and from such a viewpoint, a membrane having a hydrophilic surface is preferable. Particularly, the hydrophilic membrane is effective to suppress adsorption of proteins that give important information by proteome analysis and recover the proteins without vain. The hydrophilic membrane is not particularly limited if a membrane is made to be hydrophilic and examples of the membrane are those obtained by copolymerizing a hydrophilic monomer and a hydrophobic monomer, blending a hydrophilic polymer and a hydrophobic polymer and forming films from the blend, bonding or sticking a hydrophilic polymer to a membrane made of a hydrophobic polymer, and treating the surface of a membrane of a hydrophobic polymer by chemical treatment, plasma treatment, and radiation treatment. The chemical structure giving the hydrophilic component is provided by hydrophilic polymers, namely including, poly(vinyl pyrrolidone).

Further, if necessary, a step of concentrating proteins by removing a solvent such as water from the solution obtained by filtration by the above may be carried out.

In the concentration step, the concentration is carried out by separating and sieving using a porous film, which may be a plane filter and a hollow fiber membrane, having an effect of a molecular sieve. In the case the amount of a sample is a little, an existing commercialized concentration device comprising a plane filter attached to a tube for centrifugal separation is used and in the case the amount of a sample is a large quantity, hollow fibers are effective to be used.

The material to be used for the concentration step is not particularly limited and usable examples are materials containing one or more polymers selected from a group consisting of cellulose type polymers such as cellulose and cellulose triacetate; polycarbonates; polysulfone polymers such as polysulfones and polyether sulfones; poly(methacrylic acid) esters such as poly(methyl methacrylate); poly(acrylic acid) esters; polyamides; poly(vinylidene fluoride); polyacrylonitrile; polyesters; polyethylene; and polypropylene. Among them, polysulfones widely used in these years for dialyzers are preferable materials since the polysulfones gives membranes having an excellent fractionating property and high water permeability.

With respect to the structure of the membrane to be used in the concentration step, both of a membrane having a sponge structure, a uniform structure and an asymmetric membrane having a multilayer structure composed of a dense layer and a support layer having a high porosity and keeping the strength of the membrane may be used. With respect to the molecular fractionation capability of the membrane to be used in the concentration step, a membrane or a ultrafiltration membrane having a molecular fractionation capability of preventing peptides from passing in the physiological salt solution, for example, having an cut-off value of 0.5kDa or lower, preferably 0.1 kDa or lower is preferable to be used.

The aimed solution having a changed composition of the biological components prepared by the the invention is required to have a high ratio of proteins with a molecular weight of 15,000 or lower and as an index of the existence ratio in the inventions, $\beta 2$-microblobulin having a molecular weight of 1,1600 is employed. It is also required to contain less proteins having a high molecular weight such as albumin and immunoglobulintransferrin, and albumin having a molecular weight of around 60,000 is employed as an index. The reason for selecting albumin as the index is because, in the case of blood, the amount of albumin is highest and generally if albumin is sufficiently removed, immunoglobulin or the like having a higher molecular weight can simultaneously be removed by a method of using a membrane.

To excellently carry out proteome analysis using the obtained solution having a changed composition of biological components, the ratio of $\beta 2$-microglobulin in the total proteins of the solution having a changed composition of biological components to $\beta 2$-microglobulin in the total proteins of the biological components-containing solution is at least 10 times as high, more preferably at least 100 times as high, and even more preferably at least 1,000 times as high. That is because the amount of proteins to be injected in an apparatus such as a mass spectrometer is limited and the measurement sensitivity can be improved.

In such a manner, the solution with a low composition ratio of proteins having a molecular weight of 60,000 or higher in the total proteins can be obtained and to carry out highly sensitive analysis, the composition ratio of albumin in the proteins is desirably lower than 0.3, more desirably lower than 0.1, and even more desirably lower than 0.01.

Herein after, one examples of an element the first aspect of the invention will be described with reference to drawings.

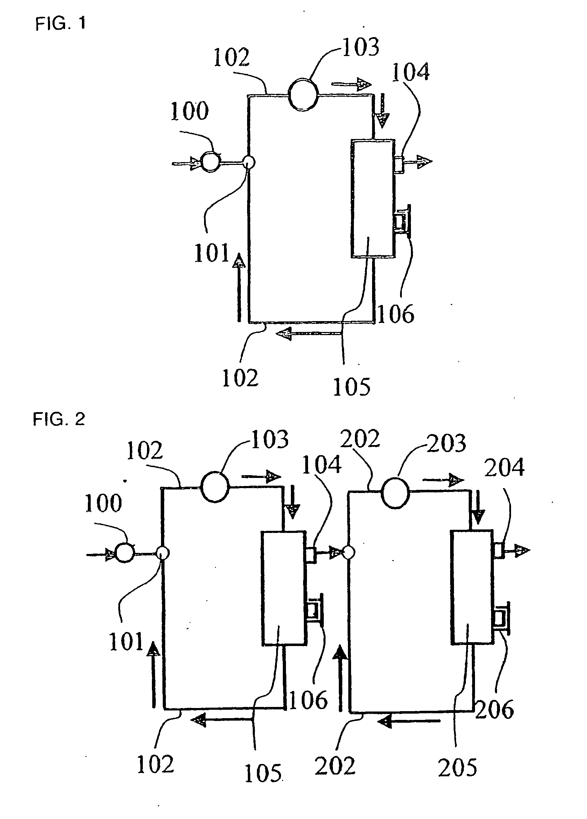

Fig. 1 is a conceptual drawing showing an example of an apparatus for preparing a solution of the first invention.

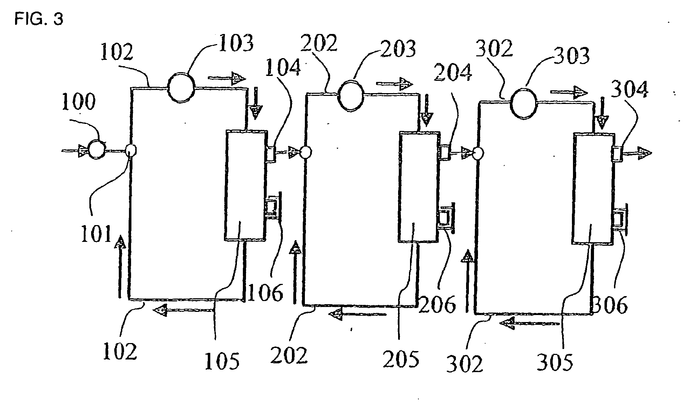

The arrow shows the flow of a solution. A sample, which is such as serum containing biological components or a biological components-containing solution containing serum is injected into a first separation membrane module 105 having a separation membrane having a specified comparative permeation ratio for the first step through a three-way valve 101 from a pump 100 for injection; sent to a solution 102 made of a tube by a circulation pump 103; and circulated there. The filtrate passed through the separation membrane in the first step is obtained in the membrane unit-treated solution recovery port 104 in the first step, which is a discharge port of the filtrate. This embodiment is a basic unit of one step. Accordingly, a desired solution for proteome analysis can be obtained. In the case it is desired to further remove proteins with a high molecular weight, a plurality of modules may be joined. Fig. 2 shows an example for treating two-step treatment by joining two modules and Fig. 3 shows an example for treating three-step treatment by joining three modules. The filtrate is injected to a unit of the next step through a tube directly joined to the treated solution recovery port of the membrane separation unit, a port for the filtrate. The aimed solution is obtained through a recovery port of a unit in the most downstream of the step (the port 104 in the apparatus of Fig. 1, the port 204 in the apparatus of Fig. 2, and the port 304 in the apparatus of Fig. 3).

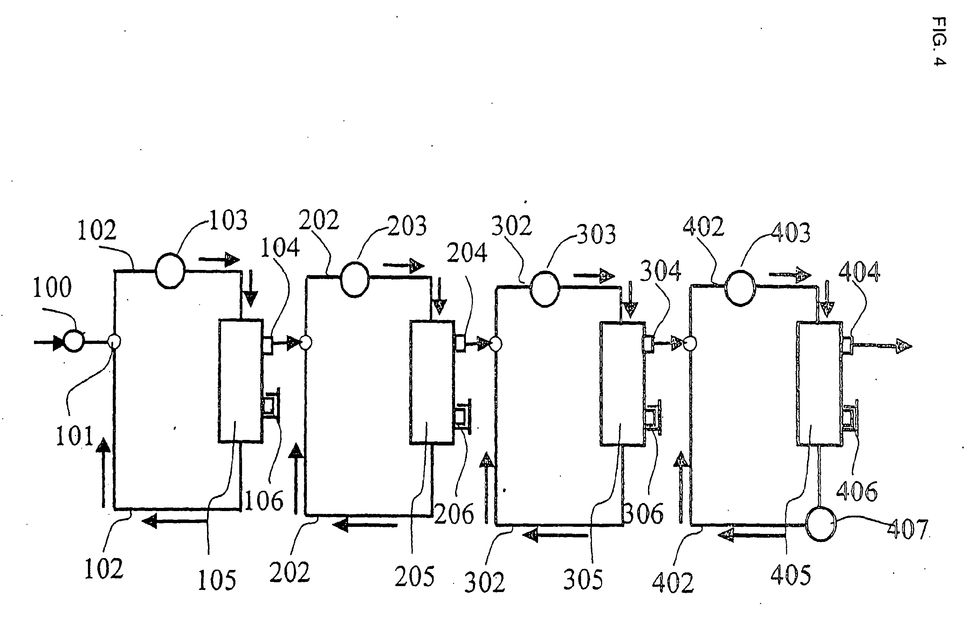

Figs. 4 shows an apparatus obtained by further comprising a further joined module having a concentration function to the apparatus of Fig. 3 and the aimed solution is recovered through a treated solution recovery port 407 of the concentration unit.

Next, a second element of an aspect of the invention will be described.

In the second element, a method of preparing a solution having a changed composition of biological components using a module containing a separation membrane by introducing a biological components-containing solution in the raw liquid side of the separation membrane, circulating the solution in the raw liquid side through a liquid circulation channel formed in the outside of the module, and taking out the solution passed through the separation membrane is characterized in that a diluting solution for the biological components-containing solution is additionally introduced into the raw liquid side of the separation membrane disposed in the module after introduction of the biological components-containing solution. Also, in this second element of the invention, the means of preparing the solution having a changed composition of biological components comprises a module containing a separation membrane disposed therein and having a raw liquid inlet and a raw liquid outlet joined to a raw liquid side flow path of the separation membrane and an outlet of the filtrate of the separation membrane; a solution circulation channel communicating the raw liquid inlet and the raw liquid outlet and having a pump and an inlet for an object solution for separation in the middle; and a diluent solution inlet formed at a position upstream of the inlet for an object solution for separation or a position in the middle of the solution.

Also in this aspect, the separation membrane is a porous separation membrane and both of a plane membrane type separation membrane such as a plane filter and a cartridge type filter and a hollow type separation membrane such as a hollow fiber membrane can be used. Particularly, the hollow fiber membrane has a high membrane surface area per unit solution treatment amount and a low pressure loss and therefore, the membrane is preferable to be used for improving the efficiency. To increase the membrane surface area per unit solution treatment amount, the inner diameter of hollow fibers is preferable to be small and it is preferably 1, 000 $\mu$m or smaller and more preferably 500 $\mu$m or smaller. The plane filter has an advantageous point that the membrane filter can be formed easily and economically. As a material for the membrane polysulfones widely used in these years for dialyzers are selected materials because of an excellent fractionating property of the polysulfones.

In the separation membrane to be used in the second element of the invention, it is necessary to use a separation membrane having a 50 or higher comparative permeation ratio of proteins having a molecular weight of less than 15, 000 and proteins having a molecular weight of 60,000 or higher. Other preferable properties and forms are same as described for the separation membrane for the first group of the inventions.

In the second element of this aspect of the invention, the invention relevant to the preparation of the solution involves introducing a biological components-containing solution into the raw liquid side of the separation membrane and thereafter introducing a diluent for the biological components-containing solution. It is because addition of the diluent solution prevents excess concentration of the biological components on the surface of the membrane and suppressed deterioration of the comparative permeation ratio of the separation membrane. Also, physiological salt solution or "a buffer solution" is preferable to be used for the diluent solution. Examples of "the buffer solution" may include MES, BIS-TRIS, ADA, ACES, PIPES, MOPSO, BIS-TRIS, PROPANE, BES, MOPS, TES, HEPES, DIPSO, MOBS, TAPSO, TRIZME, HEPPSO, POPSO, TEA, EPPS, TRICINE, GLY-GLY, BICINE, PBS, TAPS, AMPD, TABS, AMPSO, CHES, CAPSO, AMP, CAPS, AND CABS. The above-mentioned buffer solutions are abbreviated and the detailed contents can be known by referring to catalogs and MSDS (material safety data sheet) of reagent production firms such as Wako Pure Chemical Industries, Ltd. and Sigma-Aldrich Japan Co. Ltd.

The flow speed Q1 of the biological components-containing solution to be introduced into a module means a flow speed of a solution led to the module and flowing in the channel and is preferable to be higher than the flow speed Q2 of the separated solution to be sent. It is for preventing concentration of the solution in the separation membrane surface; preventing deposition of substances on the membrane surface; and preventing clogging of pores of the membrane. Further, the ratio Q2/Q1 of the flow speed Q1 of the solution led to the separation membrane module and the flow speed Q2 of the separated solution to be send is preferably 0.5 or lower. Also, in the case the ratio Q2/Q1 of the flow speed Q1 of the solution led to the separation membrane module and the flow speed Q2 of the separated solution to be send is 0, filtration is not carried out and substances are transferred only by diffusion and the separation speed is slow, so that the ratio is desirably 0.005 or higher.

The flow speed Q2 of the solution passing through the separation membrane and the flow speed Q3 of the diluent solution led to the module is desirable to satisfy the inequality of $0.5 \leq Q2/Q3 \leq 1.5$ and Q2/Q3 is preferably in a range of 0.9 to 1.1, that is around 1.

A solution from which proteins with a high molecular weight are removed and which is suitable for proteome analysis can be prepared by making two modules usable and obtaining a solution having a changed composition of biological components by additionally introducing the diluent solution as described above in a first module and obtaining a solution having a changed composition of biological components by introducing the obtained solution to a second module and simultaneously introducing the diluent solution. Further, additional third and fourth modules may be made usable and same steps may be carried out.

Based on the necessity, a step of concentrating proteins by removing a solvent such as water from a solution obtained in the above-mentioned step using one module or step using a plurality of modules may be carried out to obtain a solution having a changed composition of biological components. The materials, structure, and the capability of the membrane to be used for the concentration step are same as described in the explanation of the concentration step in the first group of the inventions.

Next, an aspect of the invention the accompanying craims will be described.

According to this aspect, a solution containing a large quantity of aimed proteins can efficiently be prepared by involving at least two steps among three steps of treating proteins by adsorption, fractionation by sieving, and concentration.

A method of preparing a solution according to this aspect of the invention is characterized in that it involves at least two steps selected from steps of (1) adsorbing proteins having a molecular weight equal to or higher than that of albumin; (2) removing a portion or all of proteins having a molecular weight equal to or higher than that of albumin by fractionation using a molecular sieve; and (3) concentrating proteins.

An apparatus for preparing a solution having a changed composition of biological components from a biological components-containing solution in this aspect of the invention comprises at least two means selected from means of (1) adsorbing a portion or all of proteins having a molecular weight equal to or higher than that of albumin; (2) removing a portion or all of proteins having a molecular weight equal to or higher than that of albumin by fractionation using a molecular sieve; and (3) concentrating proteins and the selected means are joined to each other through a flow path. The above-mentioned preferably has a flow-out path of a solution to be joined to a liquid chromatograph, an electrophoretic apparatus, or a mass spectrometer in terms of the convenience of protein analysis.

Albumin, whose molecular weight is to be a standard in this aspect of the invention includes albumin derived from human being, bovines, other mammalians, and bird, and may be determined in accordance with an object living body and in the inventions, human albumin is preferred as a standard. Proteins having a molecular weight equal to or higher than that of albumin mean mainly proteins having a molecular weight at least 60,000 to 70, 000, higher than that of albumin. Whether the molecular weight is higher than that of albumin or not can be determined by SDS-PAGE (sodium dodecyl sulphate-polyacrylamide gel electrophoresis).

Hereinafter, characteristic three steps and means of this aspect of the invention will be described.

(1) The step or means of adsorbing a portion or all of proteins with a molecular weight equal to or higher than that of albumin

Herein, "adsorption" means capturing proteins solubilized in an aqueous solution in a substance existing in the step by interaction with the substance.

A material to be used for the adsorption in this step is selected to be polysulfone type polymers such as polysulfones and polyether sulfones.

The material to be used is in form of, hollow fibers. In the respective forms, it is preferable for their surface to be roughened in order to increase the effect of the adsorption surface area. Since the material is a permeation type separation membrane, namely a hollow fiber membrane, separation of proteins with a low molecular weight can simultaneously be separated and therefore, such a material is preferable.

For the properties of the substrate itself, those whose surface is made to be hydrophilic so as not to adsorb proteins with a low molecular weight, which are needed in the aimed solution and have not to be removed, and those whose surface is made to be hydrophobic for selectively adsorbing proteins with a high molecular weight such as albumin may properly be selected and used.

The substrate having a hydrophilic surface may be of a copolymer obtained by copolymerizing a hydrophilic monomer and a hydrophobic monomer, a mixture obtained by blending a hydrophilic polymer and a hydrophobic polymer, a material obtained by bonding or sticking a hydrophilic polymer to the surface of a hydrophobic polymer, and a material obtained by treating the surface of a material of a hydrophobic polymer by chemical treatment, plasma treatment, and radiation treatment. The hydrophilic component to be used is selected from hydrophilic poly(vinyl pyrrolidone. Examples of the hydrophobic substrate to be used may be hydrophobic substances and those having a surface into which a hydrophobic ligand is introduced, more specifically polysulfones.

Further, examples to be used for the hydrophobic component may include materials in which at least one compound selected from compounds such as polyethylene imine, aminomethylpyridine, a polyphenol, a blue dye, a divalent metal ion ($Zn^{2+}$, $Ni^{2+}$, $CO^{2+}$, and $Cu^{2+}$), a compound (e.g. ethanol, isopropyl alcohol, and amidomethylated polystyrene) having a hydrophobic group (e.g. methyl, benzyl, phenyl, chloromethyl, octyl, and lauryl) is fixed.

(2) The step of removing a portion or all of proteins with a molecular weight equal to or higher than that of albumin by fractionation with a molecular sieve

In this step, it is preferable to use a porous membrane in form of a plane membrane or hollow fiber membrane and having an effect of a molecular sieve. Particularly, use of a hollow fiber membrane is effective since the surface area of the separation membrane is remarkably increased.

The material for the membrane to be used preferably in this step is polysulfone type polymers such as polysulfones and polyether sulfones. Polysulfones widely used in these years for dialyzers are preferable materials because they have an excellent fractionating property, that is a high ratio of the sieving coefficient of a substance with a low molecular weight to that of a substance with a high molecular weight among substances with different molecular weights. With respect to the membrane structure, a membrane having an asymmetric membrane having a multilayer structure composed of a dense layer and a support layer having a high porosity and keeping the strength of the membrane may be used. A conformation method of the asymmetric structure and a preferable embodiment of the asymmetric structure are same as described in the descriptions of the first group of the inventions.

As the membrane to be used for the fractionation step, there are a hydrophilic membrane and a hydrophobic membrane on the basis of the property of the membrane surface.

Examples of the hydrophilic membrane are those obtained by copolymerizing a hydrophilic monomer and a hydrophobic monomer, those obtained by blending a hydrophilic polymer and a hydrophobic polymer and forming films from the blend, those obtained by bonding or sticking a hydrophilic polymer to a membrane made of a hydrophobic polymer, and those obtained by treating the surface of a membrane of a hydrophobic polymer by chemical treatment, plasma treatment, and radiation treatment. The hydrophilic component is poly(vinyl pyrrolidone). These hydrophilic membranes can suppress adsorption of needed proteins and recover them without vain.

On the other hand, with respect to the hydrophobic membrane, those mixed with a hydrophobic component and those having a surface into which a hydrophobic ligand is introduced may be used. The hydrophobic component includes polysulfones

Further, examples to be used for the hydrophobic component may include materials in which at least one compound selected from compounds such as polyethylene imine, aminomethylpyridine, a polyphenol, a blue dye, a divalent metal ion ($Zn^{2+}$, $Ni^{2+}$, $Co^{2+}$, and $Cu^{2+}$), a compound (e.g. ethanol, isopropyl alcohol, and amidomethylated polystyrene) having a hydrophobic group (e.g. methyl, benzyl, phenyl, chloromethyl, octyl, and lauryl) is fixed or chemical reaction.

With respect to the molecular fractionation capability, membranes having a molecular fractionation capability of preventing albumin from passing in the physiological salt solution, for example, having an cut-off value of 50 kDa or lower, preferably 30 kDa or lower are preferable to be used.

In the step (1) or the step (2), various kinds of agents may be added to the aqueous solution to be loaded to improve the adsorption or fractionation capability. Practically, at least one substance among a surfactant, an emulsifier, an organic solvent, an ethylene glycol, a propylene glycol, a polyethylene imine, an aminomethylpyridine, protamine sulfate, ammonium sulfate, a polyphenol, a blue dye, a caotropic salt, and a compound (e.g. ethanol and isopropyl alcohol) having a hydrophobic group (e,g, methyl, benzyl, phenyl, chloromethyl, octyl, and lauryl) may be added.

For example, addition of a proper amount of ammonium sulfate, a polyethylene glycol, a polyethylene imine, or a chaotropic salt which promotes coagulation of albumin coagulates proteins with a high molecular weight, promotes the proteins to become huge molecules, and thus promotes adsorption of the proteins and suppresses leakage from the separation membrane, resulting in efficient prevention of permeation of the components with a high molecular weight. On the contrary, addition of a proper amount of a surfactant such as an amphoteric surfactant or an anionic surfactant suppresses interaction among proteins to efficiently carry out fractionation by molecular sieving.

(3) The step of concentrating proteins

The step of concentration means a step of concentrating proteins in a solution. Herein, the concentration step may include removing not only water from an aqueous solution but also removing components with a low molecular weight of 1 kDa or smaller. In this step, a porous membrane is used for a plane filter or a hollow fiber module to carry out concentration by separating and sieving. With respect to the molecular fractionation capability, the membrane to be used in this step differs from the separation membrane to be used in the above-mentioned fractionation step (2). Further, in the case of using the separation membrane in the above-mentioned fractionation step (2), the solution passed through the membrane is a desired solution and on the other hand, in the concentration step (3), that is different in a point that the solution remaining without being passed through the membrane is a desired solution.

In the case the amount of a sample is a little, an existing commercialized concentration device comprising a plane filter attached to a tube for centrifugal separation is used and in the case the amount of a sample is a large quantity, hollow fibers are effective to be used.

A material to be used for the separation membrane for the concentration step is one of polymers selected from cellulose type polymers such as cellulose and cellulose triacetate; polycarbonates; polysulfone type polymers such as polysulfones and polyether sulfones; poly (methacrylic acid) esters such as poly (methyl methacrylate) ; poly(acrylic acid) esters; polyamides; poly(vinylidene fluoride); polyacrylonitrile; polyesters; polyurethanes; polystyrenes; polyethylene; and polypropylene may be used. Among them, polysulfones widely used in these years for dialyzers are preferable materials because the polysulfones have an excellent fractionating property (that is, a high ratio of the sieving coefficient of a substance with a low molecular weight to that of a substance with a high molecular weight among substances with different molecular weights). With respect to the membrane structure, both a membrane having a sponge structure approximately a uniform structure and an asymmetric membrane having a multilayer structure composed of a dense layer and a support layer having a high porosity and keeping the strength of the membrane may be used. A conformation method of the asymmetric structure and a preferable embodiment of the asymmetric structure are same as described in the descriptions of the first aspect of the invention.

With respect to the molecular fractionation capability of the membrane to be used in the concentration step, a membrane or a ultrafiltration membrane having a molecular fractionation capability of preventing peptides from passing in the physiological salt solution, for example, having an cut-off value of 0.5kDa or lower, preferably 0.015 kDa or lower is preferable to be used.

This aspect of the invention is effective to make simple, automatic and continuous operation possible by joining means in the respective steps through solution flow paths and making them continuously operable. In this connection, the respective steps may be performed independently. To send a solution, a pump connected to a liquid flow path may be use and in the case of a small scale apparatus, a syringe may be used for sending a solution. In the case of concentration by using a centrifugal tube type apparatus without using a separation membrane, centrifugation operation may be carried out for the concentration.

In the method of this aspect of the invention, in the case even though a single step, the step has two or more capabilities selected from the steps of (1) adsorbing proteins with a molecular weight equal to or higher than that of albumin; (2) removing a portion or all of the proteins with a molecular weight equal to or higher than that of albumin by fractionation; and (3) concentrating proteins, a method of performing the step two or more times is also included in the scope of the inventions. Also, with respect to an apparatus of this aspect of the invention, in the case even though a single means, the means has two or more capabilities selected from the means of (1) adsorbing proteins with a molecular weight equal to or higher than that of albumin; (2) removing a portion or all of the proteins with a molecular weight equal to or higher than that of albumin by fractionation; and (3) concentrating proteins, an apparatus comprising two or more such means joined to one another is also included in the scope of the inventions.

An excellent effect to further decrease the ratio of albumin in the total amount of the proteins and increase the ratio of proteins with a low molecular weight, which are targets of analysis can be caused by repeating the same steps as the above-mentioned steps (1) to (3). Whether the same steps should be repeated or whether two different steps should be carried out can be planned in accordance with the extent of the composition of the proteins contained in the biological components to be supplied at first.

According to the preparation method disclosed in the aspect of the invention, in the case of blood plasma of a healthy adult, one time operation can cause an effect to remove proteins with a high molecular weight of 50 kDa or higher at 90% or higher removal ratio, recover proteins with a low molecular weight less than 50 kDa at 70% or higher recovery ratio, and give an average concentration ratio of the proteins with a low molecular weight less than 50 kDa at least 10 times.

Further, the one time treatment time is shortened to 1 to 6 hours. In terms of prevention of contamination with a different sample or biohazard, it is preferable to use a series of devices only one time and the apparatus to be used for the method of the inventions can be made to be disposable and it is remarkably advantageous from a viewpoint of avoidance of contamination with samples and assurance of reproducibility of analysis.

This aspect of the invention is also suitable for separating biological molecules, particularly protein components from a biological components-containing solution, particularly from human plasma, urine, saliva, tear, cerebrospinal fluid, ascites, and pleural exudate. The size of the membrane and the hollow fiber module exemplified above and the flow speed of the solution may properly be determined in accordance with the quality and the quantity of a biological material such as blood plasma and urine to be a raw material and in the case of using so-called on-table size, 1 to 400 mL, preferably 5 to 100 mL of blood plasma is used and the flow speed is adjusted to be 1 to 20 mL/min, preferably 2 to 10 mL/min.

Hereinafter, a method of changing the composition of a biological components-containing solution belonging to the third aspect of the invention and an embodiment of an apparatus for the method will be described with reference to drawings.

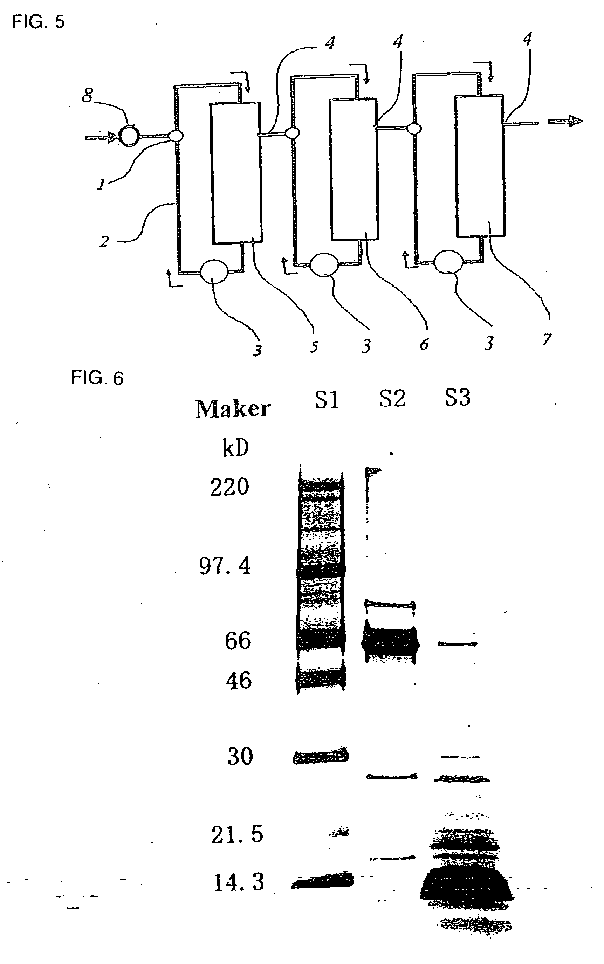

Fig. 5 is a conceptual drawing showing one example of an apparatus belonging to the third aspect of the invention and showing three steps; adsorption, fractionation, and concentration, in this order. The flow of a solution is shown with an arrow. A sample of a material such as serum or a biological components-containing solution containing the serum is injected into a module 5 of the first step having one of adsorption, fractionation, and concentration capabilities by a pump 8 for injection via a valve 1, sent to a solution circulation channel 2 made of a tube by a pump 3 and circulated. The obtained solution treated in the first step is obtained out of a treated solution 4. This performance is a unit, called a single step. Fig. 5 shows an example including three-stages and a module 6 for the second step and a module 7 for the third step are joined to each other. The obtained solution is injected to a module in the next step through a tube joined to a treated solution recovery port.

In general, in the case of a fractionation step by a molecular sieve, the outlet of the step that is a recovery port of a treated solution is an outlet of a filtrate and in the case of an adsorption step, the outlet of the step is an outlet formed in the middle of the solution circulation channel and in the case of using a permeation type membrane for the adsorption, the outlet of the filtrate becomes an outlet of the step. In the case of a concentration step, the outlet is formed in the middle of the solution circulation channel. In the embodiment shown in Fig. 5, the respective circulation pumps are formed downstream in the respective steps, however the circulation pumps may be installed upstream in the respective steps.

In the case where the concentration step is the most downstream among selected steps, treatment of the respective steps may be carried out simultaneously and treatments conventionally carried out separately while taking a long time can be carried out within a short time.

[Examples]

<Method of measuring comparative permeation ratio>

Human serum (H1388, manufactured by SIGMA or an equivalent product) is centrifuged at 3,000 rpm for 15 minutes to remove precipitates and then filtered with a 0.45 $\mu$m filter.

A separation membrane module containing a separation membrane is made ready and a raw solution (human serum) side inlet and a raw solution side (human serum) outlet are connected through a tube to form a solution circulation channel. In the middle of the solution circulation channel, a sampling port, a switch valve for discarding a solution without circulation, a flow-in path for introducing a solution into the solution circulation channel, a pump for circulating a raw solution, and a sampling port are installed along the direction from the raw solution side outlet to the raw solution side inlet. Further, a tube is connected to the filtration side outlet of the module and a pump for filtration and an outlet (a sampling port) for a filtered solution are successively formed. Through a flow-in path, the separation membrane module is filled with an aqueous PBS solution (Dulbecco PBS (-), manufactured by DAINIPPON SUMITOMO PHARMA) (hereinafter the aqueous solution is simply referred to as PBS). A raw solution, Human serum, is introduced from a raw solution side inlet at a circulation flow rate of 1 mL/min and filtration is started at a filtration flow rate of 0.2 mL/min at 20°C. During the filtration, the raw solution at the outlet of the module can be discharged without being turned back by switching the valve for discarding the solution existing in the middle of the solution circulation channel. While keeping the operation as it is from 30 minutes to 60 minutes after the start of the injection of the human serum, samples are collected from the sampling port near the raw solution inlet of the module, the sampling port near the raw solution outlet of the module, and the sampling port in the periphery of the filtration side outlet of the module. concentrations of albumin and $\beta$2-microglobulin in the respective samples are measured and the sieving coefficients of albumin and $\beta$2-microblobulin are calculated from the measured values. At that time, the average value of the concentrations of the samples collected at the respective sampling ports near the inlet and outlet of the module is employed as the raw solution side concentration. The comparative permeation ratio is defined as a value calculated by dividing the calculated sieving coefficient of $\beta$2-microglobulin by the calculated sieving coefficient of albumin. In this connection, measurement of the concentration of albumin in Examples is ordered to SRL, Inc. and carried out according to Item code 0721 4 (Latex Aggregation Immunoassay) . In the assay, those having the albumin concentration of 0.4 mg/L or lower, under the detection limit, are subjected to the measurement Human Albumin ELISA Quantitation Kit (Cat No. E80-129 ) of BETHYL Inc. Measurement of the concentration of $\beta$2-microglobulin is ordered to SRL, Inc. and carried out according to Item code 02103 (Latex Aggregation Immunoassay).

<Protein analysis by two-dimensional electrophoretic analysis>

An original biological components-containing solution and a solution having a changed composition of biological components obtained by a method of the inventions are analyzed by two-dimensional electrophoretic analysis. The method is as follows.

1. An equivalent amount of a 80% sucrose solution is added to a biological components-separated solution to obtain a sample for electrophoresis.
2. IEF-PAGE mini at pH 3 to 10 and with a thickness of 1 mm (manufactured by TEFCO Inc.), which is a commercialized isoelectric electrophoretic gel, is set in an electrophoresis tank.
3. An upper buffer (0.05 M sodium hydroxide) 200 mL and a lower buffer (0.01 M phosphoric acid) 500 mL are set.
4. The respective wells are washed with the upper buffer and 20 μL of a 10% sucrose solution is put on each well.
5. The sample prepared in (1.) is set under the sucrose solution put on each well.
6. A cover is put on the electrophoresis tank and an electric power is connected to carry out electrophoresis at 100 V for 30 minutes, at 200 V for 30 minutes, and at 500 V for 60 minutes.
7. Plastic plates of a gel cassette are peeled to take out the gel and the gel is immersed in a 40% acetic acid aqueous solution and shaken for 30 minutes.
8. The gel is dyed for 5 minutes with a dying solution of Coomassie Brilliant Blue Dye (CBB dye) and decolorized by a decolorization solution (10% methanol, 7.5% acetic acid) to observe bands and washed for 30 minutes or longer by changing water.
9. The gel is cut into one lane.
10. The cut gel piece is immersed in an electrophoresis buffer for SDS-PAGE for 10 to 20 minutes to produce equilibrium state. The composition of the electrophoresis buffer for SDS-PAGE (hereinafter referred to as electrophoresis buffer) is produced by adding distilled water to Tris 3.0 g, glycine 14.4 g, and SDS 1.0 g and adjusting the total volume to be 1000 mL.
11. A 1.5 mm thick-2-Dwell (manufacture by TEFCO Inc.) containing 4 to 20% SDS-PAGE mini, a commercialized gel for SDS-PAGE, is set in an electrophoresis tank.
12. The electrophoresis buffer is loaded and the well is washed with the electrophoresis buffer.
13. The gel piece obtained in (9.) is shifted carefully while formation of air bubbles in the 2-dimensional SDS-PAGE mini in the well is prevented. One μL of Rainbow marker (manufactured by Amersham), a commercialized molecular weight marker, is put on a small well.
14. The gel is sealed with a 1% agarose/electrophoresis buffer (Bromophenol Blue is added so slightly as to colorize the gel noticeable by eye observation at the time of preparation).
15. A cover is put of the electrophoresis tank and an electric power is connected to carry out electrophoresis at 15 mA for about 120 minutes (until Bromophenol Blue is shifted to the lower end) .
16. The gel is taken out of a gel cassette and dyed with CBB and silver dyeing. The silver dyeing is carried out by using a silver dyeing II Kit Wako, a commercialized kit, (manufactured by Wako Pure Chemical Industries, Ltd.).

(Example 1)

Polysulfone (UDEL® P-3500, manufactured by Solvay Advanced Polymers, L.L.C.) 18 part by weight and polyvinylpyrrolidone (K 30, manufactured by BASF Inc.) 9 part by weight were added to a mixed solvent of N, N' -dimethylacetamide 72 part by weight and water 1 part by weight and heated at 90°C for 14 hours for dissolution to obtain a film-formable starting solution. The film-formable starting solution was jetted out of an outside tube of a tube-in orifice type spinneret having an outer diameter of 0. 3 mm and an inner diameter of 0.2 mm. As a core solution, a solution containing N, N' -dimethylacetamide 58 part by weight and water 42 part by weight was jetted out of the inside tube. The jetted film-formable starting solution was led to 100% water after passing to a coagulation bath at a distance of 350 mm from the spinneret to obtain a hollow fiber membrane. The structure of the obtained hollow fiber membrane was observed by an electron microscope (S800, manufactured by Hitachi Ltd.) to find that the membrane had an asymmetric structure. Ten thousand hollow fiber membranes obtained as described were inserted in a cylindrical plastic case having a dialysis solution inlet and a dialysis solution outlet same as a common dialyzer and both end parts were sealed with a resin to obtain a hollow fiber membrane module having an effective membrane surface area of 1.6 6 m$^2$. After the hollow fiber membrane module was washed with water, γ-ray was radiated to the module in the state the module was filled with water. The dose of the γ-ray radiation was 27 kGy.

The hollow fiber membranes of the module were cut out and 100 membranes were bundled and both terminal ends were fixed in a module case of a glass tube with an epoxy type potting agent in a manner that the hollow parts of the hollow fiber membranes were not closed to produce a minor-module. The mini-module had an outer diameter of about 7 mm and an entire length of about 17 cm and two ports in the outside of the hollow fibers similarly to a common hollow fiber membrane type dialyzer. The hollow fiber membranes of the mini-module and the module inside were washed with distilled water.

After that, the mini-module was filled with an aqueous PBS solution (Dulbecco PBS (-), manufactured by DAINIPPON SUMITOMO PHARMA) to obtain a hollow fiber membrane mini-module (hereinafter, referred to as mini-module (1) for short). Two mini-modules were produced and the comparative permeation ratio of the separation membranes was measured by using one of them to find that the comparative permeation ratio was 70.5. The remaining mini-module was used for the following experiment.

Human serum (H 1388, Lot 28H8550, manufactured by SIGMA Inc.) was centrifuged at 3,000 rpm for 15 minutes to remove precipitate and successively filtered by a 0.45 $\mu$m filter.

One of ports in the outside of the mini-module (1) was capped and the other port was connected to a Peri-Strat™ pump via a silicone tube. On the other hand, with respect to a solution in the inside of the hollow fiber membranes, the raw solution inlet and the raw solution outlet of the module were connected with each other through a silicone tube to form a solution circulation channel and a Peri-Strat™ pump was used to circulate serum therein. Further, an inlet for additionally loading PBS was formed in a middle of the solution circulation channel.

Four mL of serum was filtered at a circulation flow rate of 5 mL/min, filtration flow rate of 0.2 mL/min, and 20°C for 4 hours (this step is the step of fractionating mainly proteins larger than albumin). The flow rate of the circulated solution was kept constant by adding PBS to the serum. The filtrate obtained for 4 hours, that is, a solution having a changed composition of biological components was 52.5 mL and had an albumin concentration of 61 mg/L, an $\alpha$1-microgloblin concentration of 0.4 mg/L, $\beta$2-microglobulin concentration of 0.066 mg/L. The total protein concentration of the used human serum was 53, 000 mg/L, the albumin concentration was 33, 000 mg/L, the $\alpha$1-microgloblin concentration was 16.5 mg/L, and $\beta$2-microgloblin concentration was 1.17 mg/L and considerable decrease of the albumin concentration was observed.

The total protein concentration in the solution was measured by Micro BCA Protein Assay (manufactured by PIERCE Inc.) and using BSA for a calibration curve. The total protein concentration in the filtrate was 275 mg/L and the ratio of the concentration of albumin to that of the total proteins was 0.22. The ratio of the $\beta$2-microglobulin concentration to the total protein concentration in the filtrate was 0.00024 while the ratio in human serum was 0.0000223 and the former was increased 10.8 times as much as the latter.

(Example 2)

Polysulfone (UDEL® P-3500, manufactured by Solvay Advanced Polymers, L.L.C.) 18 part by weight and polyvinylpyrrolidone (K 30, manufactured by BASF Inc.) 9 part by weight were added to a mixed solvent of N, N' -dimethylacetamide 72 part by weight and water 1 part by weight and heated at 90°C for 14 hours for dissolution to obtain a film-formable starting solution. The film-formable starting solution was jetted out of an outside tube of a tube-in orifice type spinneret having an outer diameter of 0. 3 mm and an inner diameter of 0.2 mm. As a core solution, a solution containing N, N' -dimethylacetamide 58 part by weight and water 42 part by weight was jetted out of the inside tube. The jetted film-formable starting solution was led to 100% water after passing to a coagulation bath at a distance of 350 mm from the spinneret to obtain a hollow fiber membrane. The structure of the obtained hollow fiber membrane was observed by an electron microscope (S800, manufactured by Hitachi Ltd.) to find that the membrane had an asymmetric structure. Ten thousand hollow fiber membranes obtained as described were inserted in a cylindrical plastic case having a dialysis solution inlet and a dialysis solution outlet same as a common dialyzer and both end parts were sealed with a resin to obtain a hollow fiber membrane module having an effective membrane surface area of 1.6 m$^2$. An aqueous solution containing 0.1% by weight of a polyethylene imine as a cationic hydrophilic polymer (weight average molecular weight 1,000,000, manufactured by BASF Inc.) was packed in the inner face side and the outer face side of the hollow fiber membranes of the hollow fiber membrane module. After that, $\gamma$-ray was radiated to the module. The dose of the $\gamma$-ray radiation was 27 kGy. The hollow fiber membranes of the module were cut out and 100 membranes were bundled and both terminal ends were fixed in a module case of a glass tube with an epoxy type potting agent in a manner that the hollow parts of the hollow fiber membranes were not closed to produce a mini-module. The mini-module had an outer diameter of about 7 mm and an entire length of about 17 cm and two ports in the outside of the hollow fibers similarly to a common hollow fiber membrane type dialyzer. The hollow fiber membranes of the mini-module and the module inside were washed with distilled water. After that, the mini-module was filled with an aqueous PBS solution (Dulbecco PBS (-), manufactured by DAINIPPON SUMITOMO PHARMA) to obtain a polyethylene imine-fixed hollow fiber membrane mini-module (hereinafter, referred to as mini-module (2) for short). Two mini-modules were produced and the comparative permeation ratio of the separation membrane was measured by using one of them to find that the comparative permeation ratio was 400. The remaining mini-module was used for the following experiment.

Human serum (H 1388, Lot 28H8550, manufactured by SIGMA Inc.) was centrifuged at 3,000 rpm for 15 minutes to remove precipitate and successively filtered by a 0.45 $\mu$m filter.

At first, a hollow fiber membrane mini-module same as that in Example 1 (the mini-module 1) was prepared and one of ports in the outside of the follow fibers was capped and the other port was connected to a silicone tube. With respect to a solution in the inside of the hollow fiber membranes, the raw solution inlet and the raw solution outlet were connected with each other through a silicone tube to form a solution circulation channel and a Peri-Strat™ pump was used to circulate a raw solution therein. A three-way valve was installed in a middle of the solution circulation channel and an injection pump was installed via a tube in one side. Further, also in the mini-module (2), one of ports in the outside of the follow fibers was capped and the other port was connected to a silicone tube. With respect to a solution in the inside of the hollow fiber membranes in this mini-module, the raw solution inlet and the raw solution outlet were connected with each other to form a solution circulation channel and a Peri-Strat™ pump was installed in a middle of the circulation channel to circulate a raw solution therein. A three-way valve was also installed in a middle of the solution circulation channel.

The silicone tube connected to the port in the outside of the mini-module (1) and the three-way valve installed in the middle of the solution circulation channel of the mini-module (2) were joined. Then, these tubes and two modules were filled with PBS to produce a system comprising two mini-modules connected to each other in series as shown in Fig. 2. Herein, use of the mini-module (1) is for a step of fractionating proteins with a molecular weight equal to or higher than albumin and use of the mini-module (2) is for both steps of adsorbing and fractionating proteins with a molecular weight equal to or higher than albumin.

Serum was loaded though tubes by the injection pump and filtration was carried out at a circulation flow rate of 5 mL/min, filtration flow rate of 0.2 mL/min, and 20°C for 4 hours in the respective solution circulation channels of the mini-modules (1) and (2). At that time, the flow rate of the circulated solution was kept constant by adding PBS in an amount equivalent to that of the filtered solution.

After 4 hours, the filtrate obtained from the module (2), a solution having a changed composition of biological components, had an albumin concentration of 0.62 mg/L, the $\alpha$1-microgloblin concentration of 0.036 mg/L, $\beta$2-microgloblin concentration of 0.05 mg/L. The total protein concentration of the used human serum was 53, 000 mg/L, the albumin concentration was 33, 000 mg/L, the $\alpha$ 1-microglobulin concentration was 16.5 mg/L, and $\beta$2-microgloblin concentration was 1.17 mg/L and considerable decrease of the albumin concentration was observed. The total protein concentration in the filtrate was 8.1 mg/L and the ratio of the concentration of albumin to that of the total proteins was 0.08. The ratio of the $\beta$2-microglobulin concentration to the total protein concentration in the filtrate was 0.00617 while the ratio in human serum was 0.0000223 and the former was increased 277 times as much as the latter.

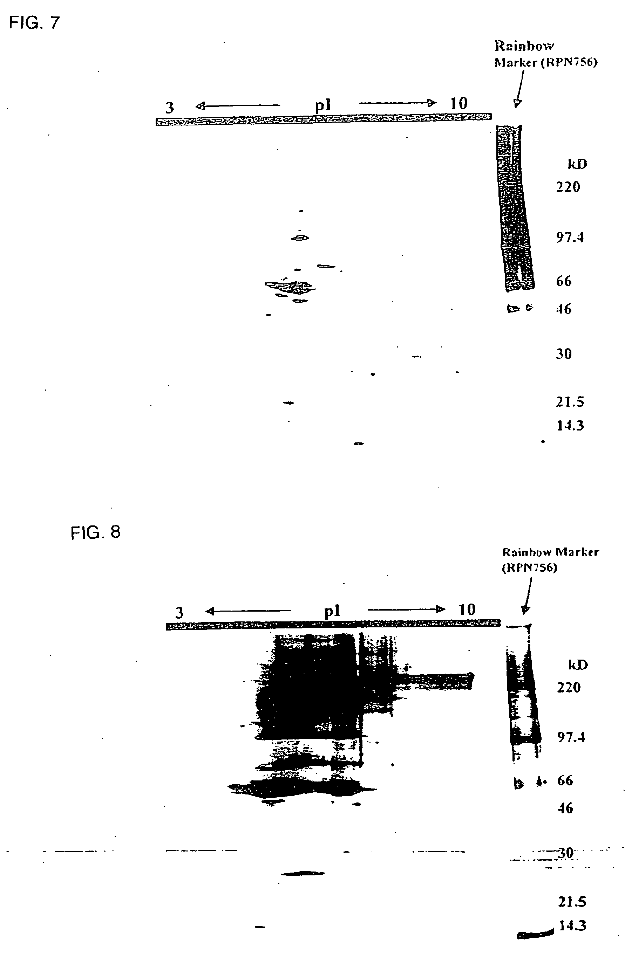

The results of the analysis of the obtained sample by two-dimensional electrophoresis are shown in Fig. 7.

Fig. 7 shows a two-dimensional electrophoresis photograph of a concentrated solution obtained using 300 $\mu$L of the solution having a changed composition of biological components produced in Example 2. As being understood from Fig. 7, many independent spots are observed in the range indicating a molecular weight of less than 60,000. The sample obtained by separation using the device has a large number of spots in a low molecular weight region.

(Comparative Example 1)

Two-dimensional electrophoretic analysis was carried out using human serum before separation as a comparative sample. Fig. 8 shows the results. Fig. 8 is a two-dimensional electrophoresis photograph of 0.5 $\mu$L of human serum before the treatment carried out in Example 2 as a sample. In Fig. 8, the spots are distributed in a broad range and substances can not be specified and at the same time, few spots are observed in a low molecular weight region.

(Example 3)

Both ends of resin adhesion portions of Dialyzer BS 1.8 1 (Lot. 20440312, manufactured by Toray Industries, Inc.) were cut to obtain hollow fiber membranes. The hollow fiber membranes had an inner diameter of 200 $\mu$m and a membrane thickness of 40 $\mu$m and the structure of the hollow fiber membranes was found asymmetric by observation of an electron microscope (S800, manufactured by Hitachi Ltd.).

The hollow fiber membranes in a number of 100 were bundled and both terminal ends were fixed in a module case of a glass tube with an epoxy type potting agent in a manner that the hollow parts of the hollow fiber membranes were not closed to produce a minor-module. The mini-module had an inner diameter of about 5 mm and an entire length of about 17 cm and two ports (blood ports) in the inside of the hollow fiber membranes and two ports (dialyzed solution ports) in the outside of the hollow fibers similarly to a common hollow fiber membrane type dialyzer. The hollow fiber membranes of the mini-module and the module inside were washed with distilled water. After that, the mini-module was filled with an aqueous PBS solution (Dulbecco PBS (-), manufactured by DAINIPPON SUMITOMO PHARMA) to obtain a hollow fiber membrane mini-module (hereinafter, referred to as mini-module (3) for short). Two mini-modules (3) were produced and the comparative permeation ratio of the separation membrane was measured by using one of them to find that the comparative permeation ratio was 149. The remaining mini-module was used for the following experiment.

Human serum (H 1388, Lot 122K0424, manufactured by SIGMA Inc.) was centrifuged at 3,000 rpm for 15 minutes to remove precipitate and successively filtered by a 0.45 $\mu$m filter.

Between the ports in the outside of the mini-module (1), the lower port 106 in the module was capped and the upper port was set to be a treated solution recovery port 104 of the membrane separation unit. The solution in the inside of the hollow fiber membranes was enabled to circulate in a solution circulation channel 102 formed by connecting the raw solution inlet and outlet through a silicone tube and installing a Peri-Strat™ pump as a circulation pump 103 in the middle. Also a three-way valve 101 was installed in the middle of the solution circulation channel 102 and an injection pump 103 is installed in one side of the three-way valve 101.

Four mL of serum was added at 1.0 mL/min by the injection pump and filtered at a circulation flow rate of 10 mL/min, filtration flow rate of 1.0 mL/min, and 20°C for 50 minutes. At that time, the flow rate of the circulated solution was kept constant by adding PBS at a low rate of 1.0 mL/min, which is the quantity equivalent to the quantity of the filtered solution passing the membranes, to the circulation channel. The amount of the filtrate solution obtained for 50 minutes was about 50 mL and the solution had an albumin concentration of 32.8 mg/L, an $\alpha$1-microgloblin concentration of 0.06 mg/L, and a $\beta$2-microgloblin concentration of 0.068 mg/L, and on the other hand, the total protein concentration of the human serum used as the raw solution was 48,000 mg/L, the albumin concentration was 29, 800 mg/L, the $\alpha$ 1-microglobulin concentration was 13.2 mg/L, and the $\beta$2-microglobulin concentration was 1.27 mg/L. The total protein amount in the human serum used as the raw solution was 192,000 $\mu$g; the albumin amount 119,000 $\mu$g; the $\alpha$1-microglobulin amount 52.8 $\mu$g; and the $\beta$2-microglobulin amount 5.08 $\mu$g and on the other hand, the total protein amount in the filtrate was 56, 000 $\mu$g; the albumin amount 1, 640 $\mu$g; the $\alpha$1-microglobulin amount 3.00 $\mu$g; and the $\beta$2-microglobulin amount 3.40 $\mu$g and accordingly, while the $\beta$2-microglobulin amount was maintained, a large quantity of albumin was removed. Based on the results, the ratio of the concentration of albumin to that of the total proteins was found to be 0.15. Also, the ratio of the $\beta$2-microglobulin concentration to the total protein concentration in the filtrate was 0.00613 while the ratio in human serum was 0.0000265 and the former was increased 23 times as much as the latter.


(Example 4 and Comparative Example 2)

One mini-module was produced using hollow fiber membranes (comparative permeation ratio 149) obtained in Example 3. The shape and the number of the mini-module were same as those of the mini-module in Example 3. Hereinafter, the mini-module is named as mini-module (4). Also, two mini-modules (hereinafter referred to as mini-module(s) (5) for short) were produced in the same manner as Example 1 by fixing 40 hollow fiber membranes (comparative permeation ratio 149) obtained in Example 3 in a module case of a glass tube with an inner diameter of about 5 mm and a length of about 12 cm. These mini-modules were used for the following experiment.

Human serum (H1388, Lot 122K0442, manufactured by SIGMA) was centrifuged at 3, 000 rpm for 15 minutes to remove precipitates and then filtered with a 0.45 $\mu$m filter.

At first, one mini-module (4) was prepared and one of outside ports was capped and the other port was connected to a silicone tube. With respect to a solution in the inside of the hollow fiber membranes, the raw solution inlet and the raw solution outlet were connected with each other through a silicone tube to form a solution circulation channel and a Peri-Strat™ pump was installed in the channel to circulate a raw solution therein. A three-way valve was installed in a middle of the solution circulation channel and an injection pump was installed in one side of the three-way valve. Further, also in one of the mini-modules (5), one of outside ports was capped and the other port was connected to a silicone tube. Also, with respect to a solution in the inside of the hollow fiber membranes in this mini-module (5), the raw solution inlet and the raw solution outlet were connected with each other to form a solution circulation channel and a Peri-Strat™ pump was installed in the channel to circulate the solution therein. A three-way valve was also installed in a middle of the solution circulation channel. The resulting mini-module was used as a membrane separation unit in the second stage. Further, one of outside ports of the other mini-module (5) was capped and the other port was connected to a silicone tube. Also, with respect to a solution in the inside of the hollow fiber membranes in this second mini-module (5), the raw solution inlet and outlet were connected with each other to form a solution circulation channel and a Peri-Strat™ pump was installed in the channel to circulate the solution therein. Further, a three-way valve was also installed in a middle of the solution circulation channel. The resulting mini-module was used as a membrane separation unit in the third stage. The silicone tubes connected to the ports in the outsides of the respective mini-modules were connected successively to the three-way valves of the next membrane separation units and PBS was loaded into the entire body of the separation system to produce the separation system comprising the membrane separation units in three stages as shown in Fig. 3.

Fig. 3 is a schematic view of the separation system used in the Example. The flow of the solution is shown with an arrow. Serum and a diluting solution (PBS) were introduced into the solution circulation channel 102 via the three-way valve 101 from the injection pump 100, further sent by the circulation pump 103, injected into the first separation membrane module 105 (the mini-module (4)) and circulated in the solution circulation channel 102. The solution obtained in the first membrane separation unit was taken out of the treated solution recovery port 104 of the membrane separation unit. Next, the solution was led to the membrane separation unit in the second stage, injected into the separation membrane module 205 in the second stage (the first mini-module (5)), and circulated in the solution circulation channel 202 in the second stage by the circulation pump 203 in the second stage. The solution passed through the separation membrane module 205 in the second stage of the second membrane separation unit was taken out of the treated solution recovery port 204. Further, the solution was led to the third membrane separation, injected into the separation membrane module 305 in the third stage (the second mini-module (5)), and circulated. The solution passed through the third separation membrane module was taken out of the treated solution recovery port 304.

The detailed conditions were as follows. After 4 mL of serum was added at 0.2 mL/min to the membrane separation unit in the first stage, filtration was carried out by circulating the solution at a flow rate of 5.0 mL/min and a filtrate flow rate of 0.2 mL/min in common in the membrane separation unit in the first stage, the membrane separation unit in the second stage, and the membrane separation unit in the third stage at 20°C for 4 hours. At that time, the amount of the circulated solution was kept constant by adding PBS in an amount equivalent to that of the filtered solution at 0.2 mL/min to the first membrane separation unit via the injection pump 100. The amount of the solution obtained for 4 hours was about 47 mL. The solution was concentrated to 4 mL by using Vivaspin 20 (3000 MWCO type), manufactured Sartorius K.K. and the concentrated solution had an albumin concentration of 0.38 mg/L, a $\beta$2-microglobulin concentration of 0.583 mg/L and on the other hand, the total protein concentration in the human serum used as the raw solution was 49,000 mg/L; the albumin concentration 31,200 mg/L; and the $\beta$2-microglobulin concentration 1.19 mg/L. While the amount of the total proteins in the human serum used as the raw solution was 196, 000 $\mu$g; the albumin amount 124,800 $\mu$g; and the $\beta$2-microglobulin amount 4.76 $\mu$g, the amount of the total proteins in the filtrate was 100 $\mu$g; the albumin amount 1.5 $\mu$g; and the $\beta$2-microgloblin amount 2.3 $\mu$g and thus, while $\beta$2-microglobulin amount was kept high, a considerable amount of albumin was removed. Based on these results, it is found that the ratio of albumin in the total proteins was 0.02. Also, the ratio of the $\beta$2-microglobulin concentration to the total protein concentration in the filtrate was 0.0307 while the ratio in human serum was 0.0000243 and the former was increased 1,263 times as much as the latter.

The sample was concentrated by using Vivaspin 500 (3000 MWCO type), manufactured Sartorius K.K. until the volume of the sample became 1/10 and an equivalent amount of a sample buffer (produced by adding a proper amount of distilled water to 0.5 M Tris-hydrochloride (pH 6.8) 0.5 mL, glycerol 0.4 m, 10% SDS 0.8 mL, and 0.1% Bromophenol Blue 0. 1 mL and adjusting the total volume to be 10 mL) was added and the resulting solution was heated for 3 minutes in a boiling water and 25 $\mu$L of the solution was subjected to the electrophoretic analysis. The results are shown in S3 lane in Fig. 6. Rainbow Marker, a commercialized molecular weight marker (RPN 756, manufactured by Amersham Biosciences) was set in S1 lane; and for comparison, a solution obtained by adding a sample buffer to the human serum (equivalent to 0.02 $\mu$L) before the treatment by the separation system of Example 4 and heated for 3 minutes in boiling water was put in S2 lane and these samples of S1 and S3 lane were simultaneously subjected to electrophoresis and the results are shown together in Fig. 6.

As being understood from the S2 lane, human serum contains a large quantity of proteins in the region of 60,000 or higher molecular weight and a small quantity of proteins in the region of 15, 000 or higher molecular weight. On the other hand, as being under stood from the S3 lane, the concentrated solution of the filtrate of Example 4 treated by the separation system contains a small quantity of proteins in the region of 60,000 or higher molecular weight and a large quantity of proteins in the region of 15,000 or higher molecular weight.

(Example 5)

The hollow fiber membranes produced in Example 1 were cut of the module before radiation of $\gamma$-ray to prepare 100 hollow fiber membranes. The hollow fiber membranes were dried at 50°C and 13% relative humidity for 24 hours. Both terminal parts of the resulting hollow fiber membranes were fixed in a module case of a glass tube with an epoxy type potting agent in the same manner as Example 4 to produce a minor-module. The hollow fiber membranes and the inside of the mini-module were washed with water and then filled with an aqueous PBS solution (Dulbecco PBS (-), manufactured by DAINIPPON SUMITOMO PHARMA) to obtain a hollow fiber membrane mini-module for concentration (hereinafter, referred to as mini-module (6)).

Further, one of ports in the outside of the mini-module (6) was capped and the other port was used as a filtrate outlet. The solution in the inside of the hollow fiber membranes was enabled to circulate in a solution circulation channel by connecting the raw solution inlet an outlet of the module were connected with each other through a silicone tube and using a Peri-Strat™ pump. Also a three-way valve was installed in a middle of the solution circulation channel. The obtained module was used as a concentration membrane unit.

A separation unit comprising three-stage membrane separation units used in Example 4 was newly produced. The treated solution recovery port of the mini-module in the third stage and the three-way valve of the concentration membrane unit were connected each other through a silicone tube. Further, a treated solution recovery port of the concentration unit comprising a three-way valve was formed in a middle of the solution circulation channel and during the concentration, only the solution circulation channel was opened. The entire body of the system was filled with PBS to produce a compounded system comprising the membrane separation unit for fractionating proteins with a molecular weight equal to or higher than that of albumin by molecular sieving and the unit for concentrating proteins bonded directly to the separation unit.

Fig. 4 shows a schematic drawing (an example of a membrane separation unit and a concentration unit) of the separation unit used in Example 5. The solution flow is shown as an arrow. Serum and a diluting solution (PBS) are injected into the solution circulation channel 102 by the injection pump 100 via the three-way valve 101, sent further by the circulation pump 103, injected into the first separation membrane module 105 (the mini-module (4)), and circulated in the solution circulation channel 102. The solution treated in the first membrane separation unit is obtained through the treated solution recovery port 104 of the membrane separation unit. Next, the solution is injected into the second separation membrane module 205 (the first mini-module (5)), and circulated in the second-stage circulation channel 202. The solution treated in the second membrane separation unit is obtained through the treated solution recovery port 204 of the membrane separation unit in the second stage. Further,

the solution is injected into the third separation membrane module 305 (the second mini-module (5)), and circulated in the third-stage circulation channel 302. The solution treated in the third membrane separation unit is obtained through the treated solution recovery port 304. Further, the solution is injected into the concentration membrane module 405 (the mini-module (6)) and circulated in the circulation channel 402 of the concentration unit. At that time, the solution passed through the concentration module is taken out of the filtrate outlet 404 of the concentration unit and discarded. On completion of the operation of the filtration and concentration, the solution remaining in the circulation channel 402 of the concentration membrane module is taken out by opening the treated solution recovery port 407 of the concentration unit.

Practical conditions were as follows. After 1 mL of serum was added at 0.2 mL/min to the membrane separation unit in the first stage, it was circulated at a flow rate of 5.0 mL/min in common in the respective solution circulation channels of the first-stage membrane separation unit, the second-stage membrane separation unit, and the third-stage membrane separation unit and filtration is carried out at a filtrate flow rate of 0.2 mL/min in common in the respective modules and 20°C for 4 hours. At that time, the amount of the circulated solution in the respective separation units and the concentration unit was kept constant by adding PBS in an amount equivalent to that of the filtered solution at 0.2 mL/min by the injection pump 100. The concentration of albumin of the solution taken through the valve 407 after 4 hour operation was 0.490 mg/L and the concentration of $\beta$2-microglobulin concentration was 0.502 mg/L and on the other hand, the total protein concentration in the human serum used as the raw solution was 49,000 mg/L; the albumin concentration 31,200 mg/L; and the $\beta$2-microglobulin concentration 1.19 mg/L. The amount of the total proteins in the human serum used as the raw solution was 196,000 $\mu$g; the albumin amount 124,800 $\mu$g; and the $\beta$2-microglobulin amount 4.76 $\mu$g, and on the other hand the amount of the filtrate was 3.7 mL, the albumin amount was 1.81 $\mu$g; and the $\beta$2-microglobulin amount was 1.86 $\mu$g and thus, while $\beta$2-microglobulin amount was kept high, a considerable amount of albumin was removed. Based on these results, it is found that the ratio of albumin in the total proteins was 0.0297. Also, the ratio of the $\beta$2-microglobulin concentration to the total protein concentration in the filtrate was 0.0304 while the ratio in human serum was 0.0000243 and the former was increased 1,253 times as much as the latter.

(Comparative Example 3)

Using a separation system comprising a commercialized plane membrane (using a polyether sulfone membrane), 4.7 mL of serum diluted to 1/4 concentration with PBS was filtered.

The concentration of albumin was 5755 mg/L and the concentration of $\beta$2-microgloblin concentration was 0.534 mg/L and on the other hand, the concentration of albumin in the human serum used as the raw solution was 128.5 mg and the concentration of $\beta$2-microglobulin was 0.446 mg/L. In the human serum used as the raw solution, the amount of albumin was 27,049 $\mu$g and the amount of $\beta$2-microglobulin was 2.51 $\mu$g, and on the other hand in the filtrate, the amount of albumin was 578 $\mu$g and the amount of $\beta$2-microglobulin amount was 2.01 $\mu$g. The comparative permeation ratio of $\beta$2-microglobulin to albumin (here, the permeation ratio was defined as the value calculated by dividing the concentration in the filtrate by the concentration in the raw solution and the comparative permeation ratio was calculated by diving the permeation ratio of $\beta$2-microglobulin by the permeation ratio of albumin) was as low as 40. The concentration ratio of albumin was as high as 0.32.

(Example 6)

A compounded system was produced in the same manner as Example 5, except that the hollow fiber membranes used in the mini-module (2) of Example 2 were used in place of the hollow fiber membranes disposed in the mini-module (6) of Example 5.

Similarly to Example 5, 1 mL of serum was treated and the solution recovered through the treated solution recovery port 407 for 4 hours had an albumin concentration of 0.3 mg/L and a $\beta$2-microgloblin of 0.515 mg/L and the concentration of the total proteins in human serum used as the raw solution was 49, 000 mg/L; the concentration of albumin was 31,200 mg/L; and the concentration of $\beta$2-microglobulin was 1.19 mg/L. The amount of the total proteins in the human serum used as the raw solution was 196,000 $\mu$g; the albumin amount 124,800 $\mu$g; and the $\beta$2-microgloblin amount 4.76 $\mu$g, and on the other hand the amount of the filtrate was 3.8 mL, the albumin amount was 1.141 $\mu$g; and the $\beta$2-microgloblin amount was 2.0 $\mu$g and thus, while $\beta$2-microglobrin amount was kept high, a considerable amount of albumin was removed. Based on these results, it is found that the ratio of albumin in the total proteins was 0.02. Also, the ratio of the $\beta$2-microgloblin concentration to the total protein concentration in the filtrate was 0.0343 while the ratio in human serum was 0.0000243 and the former was increased 1,414 times as much as the latter.

[Industrial Applicability of the Inventions]

According to a method or a preparation apparatus of the inventions, a biological components-containing solution with which analysis precision can be improved can obtained and the solution is preferable as a sample for proteome analysis in medical and pharmaceutical investigations and clinical work fields.

**Claims**

1. A method of preparing a solution for proteome analysis having a changed composition of biological components in which the concentration ratio of albumin in the total proteins is less than 0.3 and the concentration ratio of $\beta$2-microgloblin in the total proteins is at least 10 times as high as the concentration ratio in the biological components-containing starting solution, the method comprising subjecting the biological components-containing solution to treatment in at least two steps; wherein the two steps are selected from (1) a step of adsorbing a portion or all of proteins having a molecular weight equal to or higher than that of albumin; (2) a step of removing a portion or all of proteins having a molecular weight equal to or higher than that of albumin by fractionation with a molecular sieve; and (3) a step of concentrating proteins, wherein a polysulfone hollow fiber separation membrane containing flow channels having an asymmetric structure which is derived from polysulfone and poly(vinyl pyrrolidone) and has a comparative permeation ratio of $\beta$2-microglobulin to albumin, defined as

$$\frac{\texttt{sieving coefficient of } \beta\texttt{2-microglobulin}}{\texttt{sieving coefficient of albumin}},$$

of at least 50 is employed in step (1) or step (2).

2. A method according to claim 1, wherein a separation membrane containing one or more substances selected from cellulose, cellulose acetate, a polycarbonate, a polysulfone, a poly(methacrylic acid) ester, a poly(acrylic acid) ester, a polyamide, polyvinylidene fluoride, polyacrylonitrile, polyethylene, and polypropylene is used in the step (3).

3. A method according to claim 1 or 2, wherein a material fixing one or more substances selected from a group consisting of a polyethylene imine, an aminomethylpyridine, a polyphenol, a blue dye, a divalent metal ion, and an alkyl group-containing compound in the surface is used in the step (1) or the step (2).

4. A method according to any preceding claim, wherein one or more substances selected from a group consisting of a surfactant, an emulsifier, an organic solvent, an alcohol, an ethylene glycol, a propylene glycol, a polyethylene imine, an aminomethylpyridine, protamine sulfate, ammonium sulfate, a polyphenol, a blue dye, a caotropic salt, and a compound containing a hydrophobic group selected from methyl, benzyl, phenyl, chloromethyl, octyl and lauryl are added to an aqueous solution in the step (1) or the step (2).

5. A method according to any one of claims 1 to 4, wherein the biological components-containing solution contains a sample of human-derived components.

6. A method according to any preceding claim, wherein the separation membrane has a comparative permeation ratio of at least 70.

7. A method according to any preceding claim, wherein the concentration ratio of albumin in the total proteins is less than 0.1.

8. A method according to any preceding claim, wherein the membrane has a comparative permeation ratio of β2-microglobulin to albumin of no more than 10,000.

9. A method according to any preceding claim, wherein the concentration ratio of β2-microglobulin in the total proteins in the solution having a changed composition is at least 100 times as high as the concentration ratio in the biological components-containing solution.

10. A method according to any preceding claim, wherein the biological components are a solution containing proteins extracted from substances derived from blood, blood plasma, serums, urine, ascites, saliva, tear, cerebrospinal fluid, pleural exudate, or cells.

11. A method of analysis of proteins contained in biological components, which comprises preparing a solution having a changed composition of the biological components by a method as claimed in any one of claims 1 to 10 and then analyzing the proteins contained in the solution.

12. A method according to claim 11, wherein the means of analyzing proteins includes at least one selected from mass spectrometry, electrophoretic analysis, and liquid chromatography.

13. An apparatus for preparing a solution suitable for proteome analysis having a changed composition in which the concentration ratio of albumin in the total proteins is less than 0.3 and the concentration ratio of β2-microglobulin in the total proteins is at least 10 times as high as the concentration ratio in the biological components-containing starting solution, wherein the apparatus comprises at least two kinds of means joined by a flow path and selected from (1) means of adsorbing a portion or all of proteins having a molecular weight equal to or higher than that of albumin; (2) means of removing a portion or all of proteins having a molecular weight equal to or higher than that of albumin by fractionation with a molecular sieve; and (3) means of concentrating proteins, and wherein (1) or (2) comprises a polysulfone hollow fiber separation membrane containing flow channels having an asymmetric structure which is derived from polysulfone and poly(vinyl pyrrolidone) and has a comparative permeation ratio of β2-microglobulin to albumin of at least 50.

14. Apparatus according to claim 13, comprising a liquid flow-out path to be joined to a liquid chromatograph, an electrophoretic apparatus, or a mass spectrometer.

**Patentansprüche**

1. Verfahren zur Herstellung einer Lösung zur Proteomanalyse mit einer veränderten Zusammensetzung biologischer Komponenten, wobei der Konzentrationsanteil von Albumin in den gesamten Proteinen weniger als 0,3 beträgt und der Konzentrationsanteil von β2-Mikroglobulin am Gesamtprotein zumindest 10-mal höher ist als in der biologische Komponenten enthaltenden Ausgangslösung, wobei das Verfahren das Unterziehen der biologische Komponenten enthaltenen Lösung einer Behandlung mit zumindest zwei Schritten umfasst, wobei die beiden Schritte aus (1) einem Schritt zur Adsorption eines Teils der oder aller Proteine mit einem Molekulargewicht gleich jenem oder größer als jenes von Albumin; (2) einem Schritt zur Entfernung eines Teils der oder aller Proteine mit einem Molekulargewicht gleich jenem oder höher als jenes von Albumin durch Fraktionierung mittels eines Molekularsiebs; und (3) einem Schritt zur Proteinkonzentration ausgewählt sind, wobei in Schritt (1) oder Schritt (2) eine Polysulfon-Hohlfaser-Trennmembran, die Durchflusskanäle mit einer asymmetrischen Struktur aufweist und von Polysulfon und Poly(vinylpyrrolidon) abgeleitet ist, und ein komparatives Permeationsverhältnis zwischen β2-Microglobulin und Albumin, das wie folgt definiert ist:

$$\frac{\text{Siebkoeffizient von }\beta2\text{-Mikroglobulin}}{\text{Siebkoeffizient von Albumin}}$$

von zumindest 50 eingesetzt wird.

2. Verfahren nach Anspruch 1, worin in Schritt (3) eine Trennbembran eingesetzt wird, die eine oder mehrere aus Cellulose, Celluloseacetat, Polycarbonaten, Polysulfonen, Poly(methacrylsäure)estern, Polyamiden, Polyvinylidenfluoriden, Polyacrylnitril, Polyethylen und Polypropylen ausgewählte Substanzen enthält.

3. Verfahren nach Anspruch 1 oder 2, worin in Schritt (1) oder Schritt (2) ein Material eingesetzt wird, das eine oder mehrere aus der aus Polyethyleniminen, Aminomethylpyridinen, Polyphenolen, blauen Farbstoffen, zweiwertigen Metallionen und Alkylgruppen-hältigen Verbindungen bestehenden Gruppe ausgewählte Substanzen an der Oberfläche fixiert.

4. Verfahren nach einem der vorangegangenen Ansprüche, worin in Schritt (1) oder Schritt (2) eine oder mehrere Substanzen, die aus der aus Tensiden, Emulgatoren, organischen Lösungsmitteln, Alkoholen, Ethylenglykolen, Propylenglykolen, Polyethyleniminen, Aminomethylpyridinen, Protaminsulfat, Ammoniumsulfat, Polyphenolen, blauen Farbstoffen, chaotropen Salzen und Verbindungen, die eine aus Methyl, Benzyl, Phenyl, Chlormethyl, Octyl und Lauryl ausgewählte hydrophobe Gruppe enthält, bestehenden Gruppe ausgewählt sind, zu einer wässrigen Lösung zugesetzt werden.

5. Verfahren nach einem der Ansprüche 1 bis 4, worin die biologische Komponenten enthaltende Lösung eine Probe von von einem Menschen stammenden Komponenten enthält.

6. Verfahren nach einem der vorangegangenen Ansprüche, worin die Trennmembran ein komparatives Permeationsverhältnis von zumindest 70 aufweist.

7. Verfahren nach einem der vorangegangenen Ansprüche, worin der Konzentrationsanteil von Albumin am Gesamtprotein weniger als 0,1 ausmacht.

8. Verfahren nach einem der vorangegangenen Ansprüche, worin die Membran ein komparatives Permeationsverhältnis zwischen β2-Mikroglobulin und Albumin von nicht mehr als 10.000 aufweist.

9. Verfahren nach einem der vorangegangenen Ansprüche, worin der Konzentrationsanteil von β2-Mikroglobulin am Gesamtprotein in der Lösung mit einer veränderten Zusammensetzung zumindest 100-mal höher ist als der Konzentrationsanteil in der biologische Komponenten enthaltenden Lösung.

10. Verfahren nach einem der vorangegangenen Ansprüche, worin die biologischen Komponenten eine Lösung sind, die Proteine enthält, die aus Substanzen extrahiert sind, die von Blut, Blutplasma, Seren, Urin, Ascites, Speichel, Tränen, Hirn-Rückenmarksflüssigkeit, Pleuraexsudat oder Zellen stammen.

11. Verfahren zur Analyse von Proteinen, die in biologischen Komponenten enthalten sind, welches das Herstellen einer Lösung mit einer veränderten Zusammensetzung der biologischen Komponenten durch ein Verfahren nach einem der Ansprüche 1 bis 10 und das anschließende Analysieren der in der Lösung enthaltenen Proteine umfasst.

12. Verfahren nach Anspruch 11, worin das Mittel zur Analyse von Proteinen zumindest ein aus Massenspektrometrie, elektrophoretischer Analyse und Flüssigkeitschromatographie ausgewähltes umfasst.

13. Vorrichtung zur Herstellung einer zur Proteomanalyse geeigneten Lösung mit einer veränderten Zusammensetzung, worin der Konzentrationsanteil von Albumin am Gesamtprotein weniger als 0,3 beträgt und der Konzentrationsanteil von β2-Mikroglobulin am Gesamtprotein zumindest 10-mal höher ist als der Konzentrationsanteil in der biologische Komponenten enthaltenden Lösung, worin die Vorrichtung zumindest zwei Arten von Mitteln umfasst, die über einen Strömungsweg verbunden sind und aus (1) Mitteln zur Adsorption eines Teils der oder aller Proteine mit einem Molekulargewicht gleich jenem oder höher als jenes von Albumin; (2) Mitteln zur Entfernung eines Teils der oder aller Proteine mit einem Molekulargewicht gleich jenem oder höher als jenes von Albumin durch Fraktionierung mittels eines Molekularsiebs; und (3) Mitteln zur Proteinkonzentration ausgewählt sind, und worin (1) oder (2) eine Polysulfon-Hohlfaser-Trennmembran umfassen, die Durchflusskanäle mit einer asymmetrischen Struktur aufweist, von Polysulfon und Poly(vinylpyrrolidon) abgeleitet ist und ein komparatives Permeationsverhältnis zwischen β2-Microglobulin und Albumin von zumindest 50 aufweist.

14. Vorrichtung nach Anspruch 13, die einen Flüssigkeitsausflussweg umfasst, der mit einem Flüssigchromatographen, einer Elektrophoresevorrichtung oder einem Massenspektrometer zu verbinden ist.

**Revendications**

1. Procédé pour préparer une solution pour analyse de protéome, dont la composition en composants biologiques a été changée, dans laquelle le rapport de concentration d'albumine dans les protéines totales est inférieur à 0,3 et le rapport de concentration de β2-microglobuline dans les protéines totales est d'au moins 10 fois le rapport de concentration dans la solution de départ contenant des composants biologiques, le procédé consistant à soumettre la solution contenant des composants biologiques à un traitement en au moins deux étapes ; dans lequel les deux étapes sont choisies parmi (1) une étape consistant à adsorber tout ou partie des protéines ayant un poids moléculaire égal ou supérieur à celui de l'albumine ; (2) une étape consistant à éliminer tout ou partie des protéines ayant un poids moléculaire égal ou supérieur à celui de l'albumine par fractionnement avec un tamis moléculaire ; et (3) une étape consistant à concentrer les protéines, dans lequel une membrane de séparation à fibres creuses en polysulfone contenant des canaux d'écoulement ayant une structure asymétrique, qui dérive de polysulfone et de polyvinylpyrrolidone et a un rapport de perméation comparative de la β2-microglobuline à l'albumine, défini par

$$\frac{\text{coefficient de tamisage de la } \beta2\text{-microglobuline}}{\text{coefficient de tamisage de l'albumine}}$$

d'au moins 50, est employée dans l'étape (1) ou dans l'étape (2).

2. Procédé selon la revendication 1, dans lequel une membrane de séparation contenant une ou plusieurs substances choisies parmi la cellulose, l'acétate de cellulose, un polycarbonate, une polysulfone, un ester de poly(acide méthacrylique), un ester de poly(acide acrylique), un polyamide, le poly(fluorure de vinylidène), le polyacrylonitrile, le polyéthylène, et le polypropylène, est utilisée dans l'étape (3).

3. Procédé selon la revendication 1 ou 2, dans lequel un matériau fixant une ou plusieurs substances choisies dans le groupe constitué par une polyéthylène-imine, une aminométhylpyridine, un polyphénol, un colorant bleu, un ion métallique divalent, et un composé contenant un groupe alkyle dans la surface est utilisé dans l'étape (1) ou l'étape (2).

4. Procédé selon l'une quelconque des revendications précédentes, dans lequel une ou plusieurs substances choisies dans le groupe constitué par un tensioactif, un émulsionnant, un solvant organique, un alcool, un éthylèneglycol, un propylèneglycol, une polyéthylène-imine, une aminométhylpyridine, le sulfate de protamine, le sulfate d'ammonium, un polyphénol, un colorant bleu, un sel chaotropique, et un composé contenant un groupe hydrophobe choisi parmi méthyle, benzyle, phényle, chlorométhyle, octyle et lauryle, sont ajoutées à une solution aqueuse dans l'étape (1) ou l'étape (2).

5. Procédé selon l'une quelconque des revendications 1 à 4, dans lequel la solution contenant des composants biologiques contient un échantillon de composants issus d'un humain.

6. Procédé selon l'une quelconque des revendications précédentes, dans lequel la membrane de séparation a un rapport de perméation comparative d'au moins 70.

7. Procédé selon l'une quelconque des revendications précédentes, dans lequel le rapport de concentration d'albumine dans les protéines totales est inférieur à 0,1.

8. Procédé selon l'une quelconque des revendications précédentes, dans lequel la membrane a un rapport de perméation comparative de la β2-microglobuline à l'albumine non supérieur à 10 000.

9. Procédé selon l'une quelconque des revendications précédentes, dans lequel le rapport de concentration de β2-microglobuline dans les protéines totales dans la solution ayant une composition changée est d'au moins 100 fois le rapport de concentration dans la solution contenant des composants biologiques.

10. Procédé selon l'une quelconque des revendications précédentes, dans lequel les composants biologiques sont une solution contenant des protéines extraites à partir de substances dérivées de sang, de plasma sanguin, de sérum, d'urine, d'ascites, de salive, de larmes, de liquide céphalorachidien, d'exsudat pleural, ou de cellules.

**11.** Procédé d'analyse de protéines contenues dans des compositions biologiques, qui consiste à préparer une solution dont la composition en les composants biologiques a été changée par le procédé tel que revendiqué dans l'une quelconque des revendications 1 à 10 et ensuite à analyser les protéines contenues dans la solution.

**12.** Procédé selon la revendication 11, dans lequel les moyens pour analyser les protéines comprennent au moins un moyen choisi parmi une spectrométrie de masse, une analyse électrophorétique, et une chromatographie liquide.

**13.** Dispositif pour préparer une solution convenant pour une analyse de protéome dont la composition a été changée, dans laquelle le rapport de concentration d'albumine dans les protéines totales est inférieur à 0,3 et le rapport de concentration de β2-microglobuline dans les protéines totales est d'au moins 10 fois le rapport de concentration dans la solution de départ contenant des composants biologiques, lequel dispositif comprend au moins deux types de moyens joints par un trajet d'écoulement et choisis parmi (1) des moyens pour adsorber tout ou partie des protéines ayant un poids moléculaire égal ou supérieur à celui de l'albumine ; (2) des moyens pour éliminer tout ou partie des protéines ayant un poids moléculaire égal ou supérieur à celui de l'albumine par fractionnement avec un tamis moléculaire ; et (3) des moyens pour concentrer les protéines, et dans lequel (1) ou (2) comprend une membrane de séparation à fibres creuses en polysulfone contenant des canaux d'écoulement ayant une structure asymétrique, qui dérive de polysulfone et de polyvinylpyrrolidone et a un rapport de perméation comparative de la β2-microglobuline à l'albumine d'au moins 50.

**14.** Dispositif selon la revendication 13, comprenant un trajet de sortie par écoulement de liquide devant être joint à un chromatographe liquide, un dispositif électrophorétique, ou un spectromètre de masse.

FIG. 1

FIG. 2

FIG. 3

FIG. 4

FIG. 5

FIG. 6

FIG. 7

FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2002542163 A

- JP 3297707 B

### Non-patent literature cited in the description

- The human plasma proteome: history, character, and diagnostic prospects. **Anderson NL ; Anderson NG.** proteomics(Molecular & Cellular Proteomics). The American Society for Biochemistry and Molecular Biology, Inc, 2002, vol. 1, 845-867
- New Biochemical Experiments, vol. 1; Proteins (1) separation · refining · characteristics. TOKYO KAGAKUDOZIN CO., LTD, 1990, vol. 1

- Illustrated Bioexperiment 5. CELL TECHNOLOGY. SHUJUNSHA Co., Ltd, 2001
- **N. Ahmed et al.** *Proteomics,* 23 June 2003
- **D. L. Rothemund et al.** *Proteomics,* 2003, vol. 3, 279-287